(19) Europäisches Patentamt
European Patent Office
Office européen des brevets

(11) EP 1 208 186 B1

(12) **EUROPEAN PATENT SPECIFICATION**

(45) Date of publication and mention of the grant of the patent:
**10.05.2006 Bulletin 2006/19**

(21) Application number: **00958470.7**

(22) Date of filing: **16.08.2000**

(51) Int Cl.:
***C11D 3/39*** (2006.01) ***D06L 3/02*** (2006.01)
***C07D 255/02*** (2006.01)

(86) International application number:
**PCT/EP2000/008075**

(87) International publication number:
**WO 2001/016270 (08.03.2001 Gazette 2001/10)**

(54) **COMPOSITION AND METHOD FOR BLEACHING A SUBSTRATE**

ZUSAMMENSETZUNG UND VERFAHREN ZUM BLEICHEN EINES SUBSTRATS

COMPOSITION ET PROCEDE PERMETTANT LE BLANCHIMENT D'UN SUBSTRAT

(84) Designated Contracting States:
**AT BE CH CY DE DK ES FI FR GB GR IE IT LI LU MC NL PT SE**

(30) Priority: **01.09.1999 WOPCT/GB99/02876**
**01.09.1999 WOPCT/GB99/02878**
**29.02.2000 GB 0004849**

(43) Date of publication of application:
**29.05.2002 Bulletin 2002/22**

(73) Proprietors:
- **UNILEVER PLC**
  **London EC4P 4BQ (GB)**
  Designated Contracting States:
  **CY GB IE**
- **UNILEVER N.V.**
  **3013 AL Rotterdam (NL)**
  Designated Contracting States:
  **AT BE CH DE DK ES FI FR GR IT LI LU MC NL PT SE**

(72) Inventors:
- **APPEL, Adrianus, C. M.,**
  **Unilever Res. Vlardingen**
  **NL-3133 AT Vlaardingen (NL)**
- **HAGE, Ronald,**
  **Unilever Research Vlaardingen**
  **NL-3133 AT Vlaardingen (NL)**
- **TETARD, David,**
  **Unilever Research Port Sunlight**
  **Wirral,**
  **Merseyside CH63 3JW (GB)**
- **TWISKER, Robin, Stefan,**
  **Unilever Res. Vlaardingen**
  **NL-3133 AT Vlaardingen (NL)**

(74) Representative: **Elliott, Peter William et al**
**Unilever Patent Group**
**Colworth House**
**Sharnbrook**
**Bedford, MK44 1LQ (GB)**

(56) References cited:
**EP-A- 0 458 397**
**WO-A-95/28468**
**WO-A-96/06154**
**WO-A-97/38074**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 208 186 B1

## Description

### FIELD OF INVENTION

This invention relates to compositions and methods for catalytically bleaching substrates with atmospheric oxygen, more particularly using a defined class of ligand or complex as catalyst. This invention also relates to a method of treating textiles, such as laundry fabrics, using the defined class of ligand or complex as catalyst, more specifically to a method whereby bleaching by atmospheric oxygen is catalysed after the treatment.

### BACKGROUND OF INVENTION

Peroxygen bleaches are well known for their ability to remove stains from substrates. Traditionally, the substrate is subjected to hydrogen peroxide, or to substances which can generate hydroperoxyl radicals, such as inorganic or organic peroxides. Generally, these systems must be activated. One method of activation is to employ wash temperatures of 60°C or higher. However, these high temperatures often lead to inefficient cleaning, and can also cause premature damage to the substrate.

A preferred approach to generating hydroperoxyl bleach radicals is the use of inorganic peroxides coupled with organic precursor compounds. These systems are employed for many commercial laundry powders. For example, various European systems are based on tetraacetyl ethylenediamine (TAED) as the organic precursor coupled with sodium perborate or sodium percarbonate, whereas in the United States laundry bleach products are typically based on sodium nonanoyloxybenzenesulfonate (SNOBS) as the organic precursor coupled with sodium perborate.

Precursor systems are generally effective but still exhibit several disadvantages. For example, organic precursors are moderately sophisticated molecules requiring multi-step manufacturing processes resulting in high capital costs. Also, precursor systems have large formulation space requirements so that a significant proportion of a laundry powder must be devoted to the bleach components, leaving less room for other active ingredients and complicating the development of concentrated powders. Moreover, precursor systems do not bleach very efficiently in countries where consumers have wash habits entailing low dosage, short wash times, cold temperatures and low wash liquor to substrate ratios.

Alternatively, or additionally, hydrogen peroxide and peroxy systems can be activated by bleach catalysts, such as by complexes of iron and the ligand N4Py (*i.e.* N, N-bis(pyridin-2-yl-methyl)-bis(pyridin-2-yl)methylamine) disclosed in WO95/34628, or the ligand Tpen (*i.e.* N, N, N', N'-tetra(pyridin-2-yl-methyl)ethylenediamine) disclosed in WO97/48787. According to these publications, molecular oxygen may be used as the oxidant as an alternative to peroxide generating systems. However, no role in catalysing bleaching by atmospheric oxygen in an aqueous medium is reported.

It has long been thought desirable to be able to use atmospheric oxygen (air) as the source for a bleaching species, as this would avoid the need for costly hydroperoxyl generating systems. Unfortunately, air as such is kinetically inert towards bleaching substrates and exhibits no bleaching ability. Recently some progress has been made in this area. For example, WO 97/38074 reports the use of air for oxidising stains on fabrics by bubbling air through an aqueous solution containing an aldehyde and a radical initiator. A broad range of aliphatic, aromatic and heterocyclic aldehydes is reported to be useful, particularly para-substituted aldehydes such as 4-methyl-, 4-ethyl- and 4-isopropyl benzaldehyde, whereas the range of initiators disclosed includes N-hydroxysuccinimide, various peroxides and transition metal coordination complexes.

However, although this system employs molecular oxygen from the air, the aldehyde component and radical initiators such as peroxides are consumed during the bleaching process. These components must therefore be included in the composition in relatively high amounts so as not to become depleted before completion of the bleaching process in the wash cycle. Moreover, the spent components represent a waste of resources as they can no longer participate in the bleaching process.

Accordingly, it would be desirable to be able to provide a bleaching system based on atmospheric oxygen or air that does not rely primarily on hydrogen peroxide or a hydroperoxyl generating system, and that does not require the presence of organic components such as aldehydes that are consumed in the process. Moreover, it would be desirable to provide such a bleaching system that is effective in aqueous medium.

It may also be noted that the known art teaches a bleaching effect only as long as the substrate is being subjected to the bleaching treatment. Thus, there is no expectation that hydrogen peroxide or peroxy bleach systems could continue to provide a bleaching effect on a treated substrate, such as a laundry fabric after washing and drying, since the bleaching species themselves or any activators necessary for the bleaching systems would be assumed to be removed from the substrate, or consumed or deactivated, on completing the wash cycle and drying.

It would be therefore also be desirable to be able to treat a textile such that, after the treatment is completed, a bleaching effect is observed on the textile. Furthermore, it would be desirable to be able to provide a bleach treatment for textiles such as laundry fabrics whereby residual bleaching occurs when the treated fabric has been treated and is dry.

SUMMARY OF INVENTION

We have found that a selected class of ligand or complex is surprisingly effective in catalysing the bleaching of substrates using atmospheric oxygen or air. Furthermore, we have found certain novel ligands which are useful in the bleaching of substrates using atmospheric oxygen or air.

Accordingly, in a first aspect, the present invention provides a bleaching composition comprising, in an aqueous medium, atmospheric oxygen and a ligand which forms a complex with a transition metal, the complex catalysing bleaching of a substrate by the atmospheric oxygen, wherein the aqueous medium is substantially devoid of peroxygen bleach or a peroxy-based or -generating bleach system. The medium is therefore preferably insensitive or stable to catalase, which acts on peroxy species.

In a second aspect, the present invention provides a method of bleaching a substrate comprising applying to the substrate, in an aqueous medium, a ligand which forms a complex with a transition metal, the complex catalysing bleaching of the substrate by atmospheric oxygen.

Furthermore, in a third aspect, the present invention provides the use of a ligand which forms a complex with a transition metal as a catalytic bleaching agent for a substrate in an aqueous medium substantially devoid of peroxygen bleach or a peroxy-based or -generating bleach system, the complex catalysing bleaching of the substrate by the atmospheric oxygen.

We have also found that certain ligands or complexes of this class are surprisingly effective in catalysing bleaching of the substrate by atmospheric oxygen after treatment of the substrate.

Accordingly, in a fourth aspect, the present invention provides a method of treating a textile by contacting the textile with a ligand which forms a complex with a transition metal, whereby the complex catalyses bleaching of the textile by atmospheric oxygen after the treatment.

In a fifth aspect, the present invention provides a dry textile having a ligand as defined above applied or deposited thereon, whereby bleaching by atmospheric oxygen is catalysed on the textile.

Advantageously, the method according to the present invention permits all or the majority of the bleaching species in the medium (on an equivalent weight basis) to be derived from atmospheric oxygen. Thus, the-medium can be made wholly or substantially devoid of peroxygen bleach or a peroxy-based or -generating bleach system. Furthermore, the complex is a catalyst for the bleaching process and, as such, is not consumed but can continue to participate in the bleaching process. The catalytically activated bleaching system of the type in accordance with the present invention, which is based on atmospheric oxygen, is therefore both cost-effective and environmentally friendly. Moreover, the bleaching system is operable under unfavourable wash conditions which include low temperatures, short contact times and low dosage requirements. Furthermore, the method is effective in an aqueous medium and is therefore particularly applicable to bleaching of laundry fabrics. Therefore, whilst the composition and method according to the present invention may be used for bleaching any suitable substrate, the preferred substrate is a laundry fabric. The bleaching method may be carried out by simply leaving the substrate in contact with the medium for a sufficient period of time. Preferably, however, the aqueous medium on or containing the substrate is agitated.

An advantage of the method according to the fourth aspect of the invention is that, by enabling a bleaching effect even after the textile has been treated, the benefits of bleaching can be prolonged on the textile. Furthermore, since a bleaching effect is conferred to the textile after the treatment, the treatment itself, such as a laundry wash cycle, may for example be shortened. Moreover, since a bleaching effect is achieved by atmospheric oxygen after treatment of the textile, hydrogen peroxide or peroxy-based bleach systems can be omitted from the treatment substance.

The present invention also extends to a commercial package comprising a bleaching composition comprising a ligand or complex as defined below together with instructions for its use.

The present invention also extends to use of a ligand or complex as defined below in the manufacture of a bleaching composition, the bleaching composition substantially devoid of peroxygen bleach or a peroxy-based or peroxy-generating bleach system.

DETAILED DESCRIPTION OF THE INVENTION

The ligand may be present as a preformed complex of a ligand and a transition metal. Alternatively, the composition may comprise a free ligand that complexes with a transition metal already present in the water or that complexes with a transition metal present in the substrate. The composition may also be formulated as a composition of a free ligand or a transition metal-substitutable metal-ligand complex, and a source of transition metal, whereby the complex is formed *in situ* in the medium.

The ligand forms a complex with one or more transition metals, in the latter case for example as a dinuclear complex. Suitable transition metals include for example: manganese in oxidation states II-V, iron II-V, copper I-III, cobalt I-III, titanium II-IV, tungsten IV-VI, vanadium II-V and molybdenum II-VI.

The ligand forms a complex of the general formula (A1):

$$[M_aL_kX_n]Y_m \qquad (A1)$$

in which:

M represents a metal selected from Fe(II)-(III)-(IV)-(V);
L represents a ligand as herein defined, or its protonated or deprotonated analogue;
X represents a coordinating species selected from any mono, bi or tri charged anions and any neutral molecules able to coordinate the metal in a mono, bi or tridentate manner, preferably selected from $O^{2-}$, $RBO_2^{2-}$, $RCOO^-$, $RCONR^-$, $OH^-$, $NO_3^-$, NO, $S^{2-}$, $RS^-$, $PO_4^{3-}$, $PO_3OR^{3-}$, $H_2O$, $CO_3^{2-}$, $HCO_3^-$, ROH, $N(R)_3$, $ROO^-$, $O_2^{2-}$, $O_2^-$, RCN, $Cl^-$, $Br^-$, $OCN^-$, $SCN^-$, $CN^-$, $N_3^-$, $F^-$, $I^-$, $RO^-$, $ClO_4^-$, and $CF_3SO_3^-$, and more preferably selected from $O^{2-}$, $RBO_2^{2-}$, $RCOO^-$, $OH^-$, $NO_3^-$, $S^{2-}$, $RS^-$, $PO_3^{4-}$, $H_2O$, $CO_3^{2-}$, $HCO_3^-$, ROH, $N(R)_3$, $Cl^-$ , $Br^-$, $OCN^-$, $SCN^-$, RCN, $N_3^-$, $F^-$, $I^-$, $RO^-$, $ClO_4^-$, and $CF_3SO_3^-$;
Y represents any non-coordinated counter ion, preferably selected from $ClO_4^-$, $BR_4^-$ , $[MX_4]^-$ , $[MX_4]^{2-}$, $PF_6^-$, $RCOO^-$, $NO_3^-$, $RO^-$, $N^+(R)_4$, $ROO^-$, $O_2^{2-}$, $O_2^-$, $Cl^-$ , $Br^-$, $F^-$, $I^-$, $CF_3SO_3^-$, $S_2O_6^{2-}$ , $OCN^-$, $SCN^-$, $H_2O$, $RBO_2^{2-}$, $BF_4^-$ and $BPh_4^-$, and more preferably selected from $ClO_4^-$, $BR_4^-$ , $[FeCl_4]^-$, $PF_6^-$, $RCOO^-$, $NO_3^-$, $RO^-$, $N^+(R)_4$, $Cl^-$ , $Br^-$, $F^-$, $I^-$, $CF_3SO_3^-$, $S_2O_6^{2-}$ , $OCN^-$, $SCN^-$, $H_2O$ and $BF_4^-$;
a represents an integer from 1 to 10, preferably from 1 to 4;
k represents an integer from 1 to 10;
n represents an integer from 1 to 10, preferably from 1 to 4;
m represents zero or an integer from 1 to 20, preferably from 1 to 8; and
each R independently represents a group selected from hydrogen, hydroxyl, -R' and -OR', wherein R'= alkyl, alkenyl, cycloalkyl, heterocycloalkyl, aryl, heteroaryl or a carbonyl derivative group, R' being optionally substituted by one or more functional groups E, wherein E independently represents a functional group selected from -F, -Cl, -Br, -I, -OH, -OR', $-NH_2$, -NHR', $-N(R')_2$, $-N(R')_3^+$, -C(O)R', -OC(O)R', -COOH, $-COO^-$ ($Na^+$, $K^+$), -COOR', $-C(O)NH_2$, -C(O)NHR', $-C(O)N(R')_2$, heteroaryl, -R', -SR', -SH, $-P(R')_2$, $-P(O)(R')_2$, $-P(O)(OH)_2$, - $P(O)(OR')_2$, $-NO_2$, $-SO_3H$, $-SO_3^-$ ($Na^+$, $K^+$), $-S(O)_2R'$, -NHC(O)R', and -N(R')C(O)R', wherein R' represents cycloalkyl, aryl, arylalkyl, or alkyl optionally substituted by -F, -Cl, -Br, -I, $-NH_3^+$, $-SO_3H$, $-SO_3^-(Na^+, K^+)$, -COOH, $-COO^-(Na^+, K^+)$, - $P(O)(OH)_2$, or $-P(O)(O^-(Na^+, K^+))_2$, and preferably each R independently represents hydrogen, optionally substituted alkyl or optionally substituted aryl, more preferably hydrogen or optionally substituted phenyl, naphthyl or $C_{1-4}$-alkyl.

The ligand L is of the general formula (I):

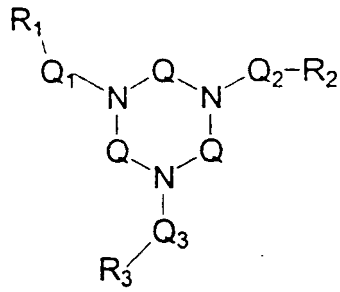

(I)

wherein
$R_1$, $R_2$, and $R_3$ independently represent a group selected from hydrogen, hydroxyl, halogen, $-NH-C(NH)NH_2$, -R and -OR, wherein R= alkyl, alkenyl, cycloalkyl, heterocycloalkyl, aryl, heteroaryl or a carbonyl derivative group, R being optionally substituted by one or more functional groups E;
Q independently represent a group selected from $C_{2-3}$-alkylene optionally substituted by H, benzyl or $C_{1-8}$-alkyl;
$Q_1$, $Q_2$ and $Q_3$ independently represent a group of the formula:

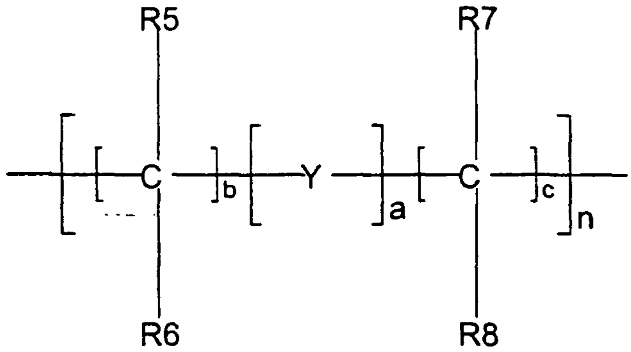

wherein
$5 \geq a+b+c \geq 1$; a=0-5; b=0-5; c=0-5; n=1 or 2;

Y independently represents a group selected from -O-, - S-, -SO-, $-SO_2-$, -C(O)-, arylene, alkylene, heteroarylene, heterocycloalkylene, -(G)P-, -P(O)- and -(G)N- , wherein G is selected from hydrogen, alkyl, aryl, arylalkyl, cycloalkyl, each except hydrogen being optionally substituted by one or more functional groups E; and

R5, R6, R7, R8 independently represent a group selected from hydrogen, hydroxyl, halogen, -R and -OR, wherein R represents alkyl, alkenyl, cycloalkyl, heterocycloalkyl, aryl, heteroaryl or a carbonyl derivative group, R being optionally substituted by one or more functional groups E,
or R5 together with R6, or R7 together with R8, or both, represent oxygen,
or R5 together with R7 and/or independently R6 together with R8, or R5 together with R8 and/or independently R6 together with R7, represent $C_{1-6}$-alkylene optionally substituted by $C_{1-4}$-alkyl, -F, -Cl, -Br or -I,
provided that at least one, preferably at least two, of $R_1$, $R_2$ and $R_3$ is a coordinating group.

At least two, and preferably at least three, of $R_1$, $R_2$ and $R_3$ independently represent a group selected from carboxylate, amido, $-NH-C(NH)NH_2$, hydroxyphenyl, an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole. Preferably, at least two of $R_1$, $R_2$, $R_3$ each independently represent a coordinating group selected from optionally substituted pyridin-2-yl, optionally substituted imidazol-2-yl, optionally substituted imidazol-4-yl, optionally substituted pyrazol-1-yl, and optionally substituted quinolin-2-yl.

Preferably, substituents for groups $R_1$, $R_2$, $R_3$, when representing a heterocyclic or heteroaromatic ring, are selected from $C_{1-4}$-alkyl, aryl, arylalkyl, heteroaryl, methoxy, hydroxy, nitro, amino, carboxyl, halo, and carbonyl.

The groups R5, R6, R7, R8 preferably independently represent a group selected from -H, hydroxy-$C_0$-$C_{20}$-alkyl, halo-$C_0$-$C_{20}$-alkyl, nitroso, formyl-$C_0$-$C_{20}$-alkyl, carboxyl-$C_0$-$C_{20}$-alkyl and esters and salts thereof, carbamoyl-$C_0$-$C_{20}$-alkyl, sulfo-$C_0$-$C_{20}$-alkyl and esters and salts thereof, sulfamoyl-$C_0$-$C_{20}$-alkyl, amino-$C_0$-$C_{20}$-alkyl, aryl-$C_0$-$C_{20}$-alkyl, $C_0$-$C_{20}$-alkyl, alkoxy-$C_0$-$C_8$-alkyl, carbonyl-$C_0$-$C_6$-alkoxy, and $C_0$-$C_{20}$-alkylamide. Preferably, none of R6-R8 is linked together. Preferably, $Q_1$, $Q_2$ and $Q_3$ are defined such that a=b=0, c=1,2,3 or 4 and n=1. Preferably, the groups $Q_1$, $Q_2$ and $Q_3$ independently represent a group selected from $-CH_2-$ and $-CH_2CH_2-$.

Group Q is preferably a group selected from $-CH_2CH_2-$ and $-CH_2CH_2CH_2-$.

In a first preferred embodiment, the ligand L is of the general formula (II):

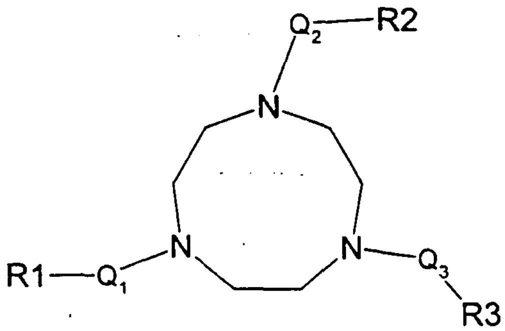

(II)

wherein R1, R2, R3 are as defined previously for $R_1$, $R_2$, $R_3$, and $Q_1$, $Q_2$, $Q_3$ are as defined previously.

Preferred classes of ligands according to the first preferred embodiment, as represented by formula (II) above, are as follows:

(i) ligands of the general formula (II) wherein:

R1, R2, R3 each independently represent a coordinating group selected from carboxylate, amido, -NH-C(NH)$NH_2$, hydroxyphenyl, an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole.

In this class, we prefer that:

R1, R2, R3 each independently represent a coordinating group selected from optionally substituted pyridin-2-yl, optionally substituted imidazol-2-yl, optionally substituted imidazol-4-yl, optionally substituted pyrazol-1-yl, and optionally substituted quinolin-2-yl.

(ii) ligands of the general formula (II) wherein:

two of R1, R2, R3 each independently represent a coordinating group selected from carboxylate, amido, -NH-C(NH)$NH_2$, hydroxyphenyl, an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole; and
one of R1, R2, R3 represents a group selected from hydrogen, $C_{1-20}$ optionally substituted alkyl, $C_{1-20}$ optionally substituted arylalkyl, aryl, and $C_{1-20}$ optionally substituted $NR_3^+$ (wherein R=$C_{1-8}$-alkyl).

In this class, we prefer that:

two of R1, R2, R3 each independently represent a coordinating group selected from optionally substituted pyridin-2-yl, optionally substituted imidazol-2-yl, optionally substituted imidazol-4-yl, optionally substituted pyrazol-1-yl, and optionally substituted quinolin-2-yl; and
one of R1, R2, R3 represents a group selected from hydrogen, $C_{1-10}$ optionally substituted alkyl, $C_{1-5}$-furanyl, $C_{1-5}$ optionally substituted benzylalkyl, benzyl, $C_{1-5}$ optionally substituted alkoxy, and $C_{1-20}$ optionally substituted $N^+Me_3$.

In especially preferred embodiments, the ligand L is selected from:

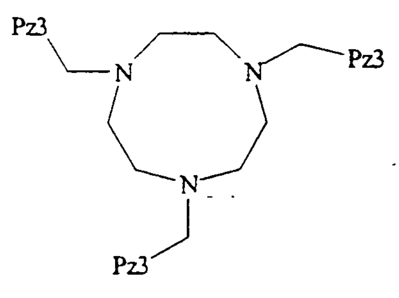

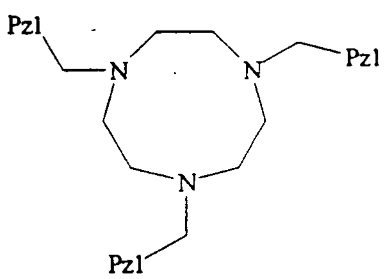

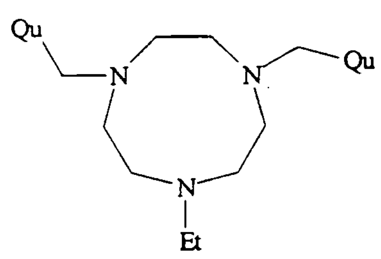

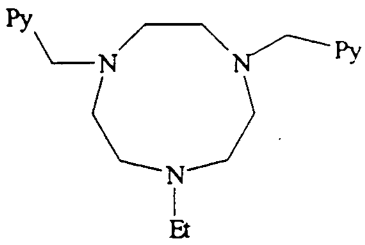

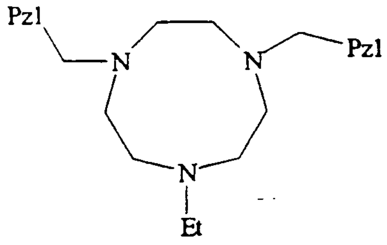

wherein -Et represents ethyl, -Py represents pyridin-2-yl, Pz3 represents pyrazol-3-yl, Pz1 represents pyrazol-1-yl, and Qu represents quinolin-2-yl.

The counter ions Y in formula (A1) balance the charge z on the complex formed by the ligand L, metal M and coordinating species X. Thus, if the charge z is positive, Y may be an anion such as $RCOO^-$, $BPh_4^-$, $ClO_4^-$, $BF_4^-$, $PF_6^-$, $RSO_3^-$, $RSO_4^-$, $SO_4^{2-}$ , $NO_3^-$, $F^-$, $Cl^-$, $Br^-$, or $I^-$, with R being hydrogen, optionally substituted alkyl or optionally substituted aryl. If z is negative, Y may be a common cation such as an alkali metal, alkaline earth metal or (alkyl)ammonium cation.

Suitable counter ions Y include those which give rise to the formation of storage-stable solids. Preferred counter ions for the preferred metal complexes are selected from $R^7COO^-$, $ClO_4^-$, $BF_4^-$, $PF_6^-$, $RSO_3^-$ (in particular $CF_3SO_3^-$), $RSO_4^-$, $SO_4^{2-}$ , $NO_3^-$, $F^-$, $Cl^-$, $Br^-$, and $I^-$, wherein R represents hydrogen or optionally substituted phenyl, naphthyl or $C_1$-$C_4$ alkyl.

It will be appreciated that the complex (A1) can be formed by any appropriate means, including *in situ* formation whereby precursors of the complex are transformed into the active complex of general formula (A1) under conditions of storage or use. Preferably, the complex is formed as a well-defined complex or in a solvent mixture comprising a salt of the metal M and the ligand L or ligand L-generating species. Alternatively, the catalyst may be formed *in situ* from suitable precursors for the complex, for example in a solution or dispersion containing the precursor materials. In one such example, the active catalyst may be formed in situ in a mixture comprising a salt of the metal M and the ligand L, or a ligand L-generating species, in a suitable solvent. Thus, for example, if M is iron, an iron salt such as $FeSO_4$ can be mixed in solution with the ligand L, or a ligand L-generating species, to form the active complex. Thus, for example, the composition may formed from a mixture of the ligand L and a metal salt $MX_n$ in which preferably n=1-5, more preferably 1-3. In another such example, the ligand L, or a ligand L-generating species, can be mixed with metal M ions present in the substrate or wash liquor to form the active catalyst *in situ*. Suitable ligand L-generating species include metal-free compounds or metal coordination complexes that comprise the ligand L and can be substituted by metal M ions to form the active complex according the formula (A1).

The bleaching compositions according to the present invention may be used for laundry cleaning, hard surface cleaning (including cleaning of lavatories, kitchen work surfaces, floors, mechanical ware washing etc.). As is generally known in the art, bleaching compositions are also employed in waste-water treatment, pulp bleaching during the manufacture of paper, leather manufacture, dye transfer inhibition, food processing, starch bleaching, sterilisation, whitening-in oral hygiene preparations and/or contact lens disinfection.

In the context of the present invention bleaching should be understood as relating generally to the decolourisation of stains or of other materials attached to or associated with a substrate. However, it is envisaged that the present invention can be applied where a requirement is the removal and/or neutralisation by an oxidative bleaching reaction of malodours or other undesirable components attached to or otherwise associated with a substrate. Furthermore, in the context of the present invention bleaching is to be understood as being restricted to any bleaching mechanism or process that does not require the presence of light or activation by light. Thus, photobleaching compositions and processes relying on the use of photobleach catalysts or photobleach activators and the presence of light are excluded from the present invention.

In typical washing compositions the level of the catalyst is such that the in-use level is from 1$\mu$M to 50mM, with preferred in-use levels for domestic laundry operations falling in the range 10 to 100 $\mu$M. Higher levels may be desired and applied in industrial bleaching processes, such as textile and paper pulp bleaching.

Preferably, the aqueous medium has a pH in the range from pH 6 to 13, more preferably from pH 6 to 11, still more preferably from pH 8 to 11, and most preferably from pH 8 to 10, in particular from pH 9 to 10.

The bleaching composition of the present invention has particular application in detergent formulations, especially for laundry cleaning. Accordingly, in another preferred embodiment, the present invention provides a detergent bleach composition comprising a bleaching composition as defined above and additionally a surface-active material, optionally together with detergency builder.

The bleach composition according to the present invention may for example contain a surface-active material in an amount of from 10 to 50% by weight. The surface-active material may be naturally derived, such as soap, or a synthetic material selected from anionic, nonionic, amphoteric, zwitterionic, cationic actives and mixtures thereof. Many

suitable actives are commercially available and are fully described in the literature, for example in "Surface Active Agents and Detergents", Volumes I and II, by Schwartz, Perry and Berch.

Typical synthetic anionic surface-actives are usually watersoluble alkali metal salts of organic sulfates and sulfonates having alkyl groups containing from about 8 to about 22 carbon atoms, the term "alkyl" being used to include the alkyl portion of higher aryl groups. Examples of suitable synthetic anionic detergent compounds are sodium and ammonium alkyl sulfates, especially those obtained by sulfating higher ($C_8$-$C_{18}$) alcohols produced, for example, from tallow or coconut oil; sodium and ammonium alkyl ($C_{9-}C_{20}$) benzene sulfonates, particularly sodium linear secondary alkyl ($C_{10}$-$C_{15}$) benzene sulfonates; sodium alkyl glyceryl ether sulfates, especially those ethers of the higher alcohols derived from tallow or coconut oil fatty acid monoglyceride sulfates and sulfonates; sodium and ammonium salts of sulfuric acid esters of higher ($C_9$-$C_{18}$) fatty alcohol alkylene oxide, particularly ethylene oxide, reaction products; the reaction products of fatty acids such as coconut fatty acids esterified with isethionic acid and neutralised with sodium hydroxide; sodium and ammonium salts of fatty acid amides of methyl taurine; alkane monosulfonates such as those derived by reacting alpha-olefins ($C_8$-$C_{20}$) with sodium bisulfite and those derived by reacting paraffins with $SO_2$ and $Cl_2$ and then hydrolysing with a base to produce a random sulfonate; sodium and ammonium ($C_7$-$C_{12}$) dialkyl sulfosuccinates; and olefin sulfonates, which term is used to describe material made by reacting olefins, particularly ($C_{10}$-$C_{20}$) alpha-olefins, with $SO_3$ and then neutralising and hydrolysing the reaction product. The preferred anionic detergent compounds are sodium ($C_{10}$-$C_{15}$) alkylbenzene sulfonates, and sodium ($C_{16}$-$C_{18}$) alkyl ether sulfates.

Examples of suitable nonionic surface-active compounds which may be used, preferably together with the anionic surface-active compounds, include, in particular, the reaction products of alkylene oxides, usually ethylene oxide, with alkyl ($C_6$-$C_{22}$) phenols, generally 5-25 EO, i.e. 5-25 units of ethylene oxides per molecule; and the condensation products of aliphatic ($C_8$-$C_{18}$) primary or secondary linear or branched alcohols with ethylene oxide, generally 2-30 EO. Other so-called nonionic surface-actives include alkyl polyglycosides, sugar esters, long-chain tertiary amine oxides, long-chain tertiary phosphine oxides and dialkyl sulfoxides.

Amphoteric or zwitterionic surface-active compounds can also be used in the compositions of the invention but this is not normally desired owing to their relatively high cost. If any amphoteric or zwitterionic detergent compounds are used, it is generally in small amounts in compositions based on the much more commonly used synthetic anionic and nonionic actives.

The detergent bleach composition of the invention will preferably comprise from 1 to 15 % wt of anionic surfactant and from 10 to 40 % by weight of nonionic surfactant. In a further preferred embodiment, the detergent active system is free from $C_{16}$-$C_{12}$ fatty acid soaps.

The bleach composition of the present invention may also contains a detergency builder, for example in an amount of from about 5 to 80 % by weight, preferably from about 10 to 60 % by weight.

Builder materials may be selected from 1) calcium sequestrant materials, 2) precipitating materials, 3) calcium ion-exchange materials and 4) mixtures thereof.

Examples of calcium sequestrant builder materials include alkali metal polyphosphates, such as sodium tripolyphosphate; nitrilotriacetic acid and its watersoluble salts; the alkali metal salts of carboxymethyloxy succinic acid, ethylene diamine tetraacetic acid, oxydisuccinic acid, mellitic acid, benzene polycarboxylic acids, citric acid; and polyacetal carboxylates as disclosed in US-A-4,144,226 and US-A-4,146,495.

Examples of precipitating builder materials include sodium orthophosphate and sodium carbonate.

Examples of calcium ion-exchange builder materials include the various types of water-insoluble crystalline or amorphous aluminosilicates, of which zeolites are the best known representatives, e.g. zeolite A, zeolite B (also known as zeolite P), zeolite C, zeolite X, zeolite Y and also the zeolite P-type as described in EP-A-0,384,070.

In particular, the compositions of the invention may contain any one of the organic and inorganic builder materials, though, for environmental reasons, phosphate builders are preferably omitted or only used in very small amounts. Typical builders usable in the present invention are, for example, sodium carbonate, calcite/carbonate, the sodium salt of nitrilotriacetic acid, sodium citrate, carboxymethyloxy malonate, carboxymethyloxy succinate and water-insoluble crystalline or amorphous aluminosilicate builder materials, each of which can be used as the main builder, either alone or in admixture with minor amounts of other builders or polymers as co-builder.

It is preferred that the composition contains not more than 5% by weight of a carbonate builder, expressed as sodium carbonate, more preferably not more than 2.5 % by weight to substantially nil, if the composition pH lies in the lower alkaline region of up to 10.

Apart from the components already mentioned, the bleach composition of the present invention can contain any of the conventional additives in amounts of which such materials are normally employed in fabric washing detergent compositions. Examples of these additives include buffers such as carbonates, lather boosters, such as alkanolamides, particularly the monoethanol amides derived from palmkernel fatty acids and coconut fatty acids; lather depressants, such as alkyl phosphates and silicones; anti-redeposition agents, such as sodium carboxymethyl cellulose and alkyl or substituted alkyl cellulose ethers; stabilisers, such as phosphonic acid derivatives (*i.e.* Dequest® types); fabric softening agents; inorganic salts and alkaline buffering agents, such as sodium sulfate and sodium silicate; and, usually in very

small amounts, fluorescent agents; perfumes; enzymes, such as proteases, cellulases, lipases, amylases and oxidases; germicides and colourants.

Transition metal sequestrants such as EDTA, and phosphonic acid derivatives such as EDTMP (ethylene diamine tetra(methylene phosphonate)) may also be included, in addition to the ligand specified, for example to improve the stability sensitive ingredients such as enzymes, fluorescent agents and perfumes, but provided the composition remains bleaching effective. However, the composition according to the present invention containing the ligand, is preferably substantially, and more preferably completely, devoid of transition metal sequestrants (other than the ligand).

Whilst the present invention is based on the catalytic bleaching of a substrate by atmospheric oxygen or air, it will be appreciated that small amounts of hydrogen peroxide or peroxy-based or -generating systems may be included in the composition, if desired. Therefore, by "substantially devoid of peroxygen bleach or peroxy-based or -generating bleach systems" is meant that the composition contains from 0 to 50 %, preferably from 0 to 10 %, more preferably from 0 to 5. %, and optimally from 0 to 2 % by molar weight on an oxygen basis, of peroxygen bleach or peroxy-based or -generating bleach systems. Preferably, however, the composition will be wholly devoid of peroxygen bleach or peroxy-based or -generating bleach systems.

Thus, at least 10 %, preferably at least 50 % and optimally at least 90 % of any bleaching of the substrate is effected by oxygen sourced from the air.

According to the fourth aspect, the catalyst may be contacted to the textile fabric in any suitable manner. For example, it may be applied in dry form, such as in powder form, or in a liquor that is then dried, for example as an aqueous spray-on fabric treatment fluid or a wash liquor for laundry cleaning, or a non-aqueous dry cleaning fluid or spray-on aerosol fluid. Other suitable means of contacting the catalyst to the textile may be used, as further explained below.

Any suitable textile that is susceptible to bleaching or one that one might wish to subject to bleaching may be used. Preferably the textile is a laundry fabric or garment.

The bleaching method of the fourth aspect may be carried out by simply leaving the substrate in contact with the catalyst for a sufficient period of time. Preferably, however, the catalyst is in an aqueous medium, and the aqueous medium on or containing the substrate is agitated.

In a preferred embodiment, the treated textile is dried, by allowing it to dry under ambient temperature or at elevated temperatures.

In a particularly preferred embodiment the method according to the fourth aspect is carried out on a laundry fabric using aqueous treatment liquor. In particular the treatment may be effected in, or as an adjunct to, an essentially conventional wash cycle for cleaning laundry. More preferably, the treatment is carried out in an aqueous detergent wash liquor. The catalyst can be delivered into the wash liquor from a powder, granule, pellet, tablet, block, bar or other such solid form. The solid form can comprise a carrier, which can be particulate, sheet-like or comprise a three-dimensional object. The carrier can be dispersible or soluble in the wash liquor or may remain substantially intact. In other embodiments, the catalyst can be delivered into the wash liquor from a paste, gel or liquid concentrate.

It is particularly advantageous that the catalyst used in the method of the fourth aspect makes use of atmospheric oxygen in its bleaching activity. This avoids the requirement that peroxygen bleaches and/or other relatively large quantities of reactive substances need be used in the treatment process. Consequently, only a relatively small quantity of bleach active substance need be employed and this allows dosage routes to be exploited which could previously not be used. Thus, while it is preferable to include the catalyst in a composition that is normally used in a washing process, such as a pre-treatment, main-wash, conditioning composition or ironing aid, other means for ensuring that the catalyst is present in the wash liquor may be envisaged. For example, it is envisaged that the catalyst can be presented in the form of a body from which it is slowly released during the whole or part of the laundry process. Such release can occur over the course of a single wash or over the course of a plurality of washes. In the latter case it is envisaged that the catalyst can be released from a carrier substrate used in association with the wash process, e.g. from a body placed in the dispenser drawer of a washing machine, elsewhere in the delivery system or in the drum of the washing machine. When used in the drum of the washing machine the carrier can be freely moving or fixed relative to the drum. Such fixing can be achieved by mechanical means, for example by barbs that interact with the drum wall, or employ other forces, for example a magnetic force. The modification of a washing machine to provide for means to hold and retain such a carrier is envisaged similar means being known from the analogous art of toilet block manufacture. Freely moving carriers such as shuttles for dosage of surfactant materials and/or other detergent ingredients into the wash can comprise means for the release of the catalyst into the wash.

In the alternative, the catalyst can be presented in the form of a wash additive that preferably is soluble. The additive can take any of the physical forms used for wash additives, including powder, granule, pellet, sheet, tablet, block, bar or other such solid form or take the form of a paste, gel or liquid. Dosage of the additive can be unitary or in a quantity determined by the user. While it is envisaged that such additives can be used in the main washing cycle, the use of them in the conditioning or drying cycle is not hereby excluded.

The present invention is not limited to those circumstances in which a washing machine is employed, but can

be applied where washing is performed in some alternative vessel. In these circumstances it is envisaged that the catalyst can be delivered by means of slow release from the bowl, bucket or other vessel which is being employed, or from any implement which is being employed, such as a brush, bat or dolly, or from any suitable applicator.

Suitable pre-treatment means for application of the catalyst to the textile material prior to the main wash include sprays, pens, roller-ball devices, bars, soft solid applicator sticks and impregnated cloths or cloths containing microcapsules. Such means are well known in the analogous art of deodorant application and/or in spot treatment of textiles. Similar means for application are employed in those embodiments where the catalyst is applied after the main washing and/or conditioning steps have been performed, e.g. prior to or after ironing or drying of the cloth. For example, the catalyst may be applied using tapes, sheets or sticking plasters coated or impregnated with the substance, or containing microcapsules of the substance. The catalyst may for example be incorporated into a drier sheet so as to be activated or released during a tumble-drier cycle, or the substance can be provided in an impregnated or microcapsule-containing sheet so as to be delivered to the textile when ironed.

Throughout the description and claims generic groups have been used, for example alkyl, alkoxy, aryl. Unless otherwise specified the following are preferred group restrictions that may be applied to generic groups found within compounds disclosed herein:

alkyl: linear and branched C1-C8-alkyl,

alkenyl: C2-C6-alkenyl,

cycloalkyl: C3-C8-cycloalkyl,

alkoxy: C1-C6-alkoxy,

alkylene: selected from the group consisting of: methylene; 1,1-ethylene; 1,2-ethylene; 1,1-propylidene; 1,2-propylene; 1,3-propylene; 2,2-propylidene; butan-2-ol-1,4-diyl; propan-2-ol-1,3-diyl; 1,4-butylene; cyclohexane-1,1-diyl; cyclohexan-1,2-diyl; cyclohexan-1,3-diyl; cyclohexan-1,4-diyl; cyclopentane-1,1-diyl; cyclopentan-1,2-diyl; and cyclopentan-1,3-diyl,

aryl: selected from homoaromatic compounds having a molecular weight under 300,

arylene: selected from the group consisting of: 1,2-phenylene; 1,3-phenylene; 1,4-phenylene; 1,2-naphtalenylene; 1,3-naphtalenylene; 1,4-naphtalenylene; 2,3-naphtalenylene; 1-hydroxy-2,3-phenylene; 1-hydroxy-2,4-phenylene; 1-hydroxy-2,5-phenylene; and 1-hydroxy-2,6-phenylene,

heteroaryl: selected from the group consisting of: pyridinyl; pyrimidinyl; pyrazinyl; triazolyl; pyridazinyl; 1,3,5-triazinyl; quinolinyl; isoquinolinyl; quinoxalinyl; imidazolyl; pyrazolyl; benzimidazolyl; thiazolyl; oxazolidinyl; pyrrolyl; carbazolyl; indolyl; and isoindolyl, wherein the heteroaryl may be connected to the compound via any atom in the ring of the selected heteroaryl,

heteroarylene: selected from the group consisting of: pyridindiyl; quinolindiyl; pyrazodiyl; pyrazoldiyl; triazolediyl; pyrazindiyl; and imidazolediyl, wherein the heteroarylene acts as a bridge in the compound via any atom in the ring of the selected heteroarylene, more specifically preferred are: pyridin-2,3-diyl; pyridin-2,4-diyl; pyridin-2,5-diyl; pyridin-2,6-diyl; pyridin-3,4-diyl; pyridin-3,5-diyl; quinolin-2,3-diyl; quinolin-2,4-diyl; quinolin-2,8-diyl; isoquinolin-1,3-diyl; isoquinolin-1,4-diyl; pyrazol-1,3-diyl; pyrazol-3,5-diyl; triazole-3,5-diyl; triazole-1,3-diyl; pyrazin-2,5-diyl; and imidazole-2,4-diyl,

heterocycloalkyl: selected from the group consisting of: pyrrolinyl; pyrrolidinyl; morpholinyl; piperidinyl; piperazinyl; hexamethylene imine; 1,4-piperazinyl; tetrahydrothiophenyl; tetrahydrofuranyl; 1,4,7-triazacyclononanyl; 1,4,8,11-tetraazacyclotetradecanyl; 1,4,7,10,13-pentaazacyclopentadecanyl; 1,4-diaza-7-thia-cyclononanyl; 1,4-diaza-7-oxa-cyclononanyl; 1,4,7,10-tetraazacyclododecanyl; 1,4-dioxanyl; 1,4,7-trithia-cyclononanyl; tetrahydropyranyl; and oxazolidinyl, wherein the heterocycloalkyl may be connected to the compound via any atom in the ring of the selected heterocycloalkyl,

heterocycloalkylene: selected from the group consisting of: piperidin-1,2-ylene; piperidin-2,6-ylene; piperidin-4,4-ylidene; 1,4-piperazin-1,4-ylene; 1,4-piperazin-2,3-ylene; 1,4-piperazin-2,5-ylene; 1,4-piperazin-2,6-ylene; 1,4-piperazin-1,2-ylene; 1,4-piperazin-1,3-ylene; 1,4-piperazin-1,4-ylene; tetrahydrothiophen-2,5-ylene; tetrahydrothiophen-3,4-ylene; tetrahydrothiophen-2,3-ylene; tetrahydrofuran-2,5-ylene; tetrahydrofuran-3,4-ylene; tetrahydrofuran-2,3-ylene; pyrrolidin-2,5-ylene; pyrrolidin-3,4-ylene; pyrrolidin-2,3-ylene; pyrrolidin-1,2-ylene; pyrrolidin-1,3-ylene; pyrrolidin-2,2-ylidene; 1,4,7-triazacyclonon-1,4-ylene; 1,4,7-triazacyclonon-2,3-ylene; 1,4,7-triazacyclonon-2,9-ylene; 1,4,7-triazacyclonon-3,8-ylene; 1,4,7-triazacyclonon-2,2-ylidene; 1,4,8,11-tetraazacyclotetradec-1,4-ylene; 1,4,8,11-tetraazacyclotetradec-1,8-ylene; 1,4,8,11-tetraazacyclotetradec-2,3-ylene; 1,4,8,11-tetraazacyclotetradec-2,5-ylene; 1,4,8,11-tetraazacyclotetradec-1,2-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-1,2-ylene; 1,4,7,10-tetraazacyclododec-2,3-ylene; 1,4,7,10-tetraazacyclododec-2,2-ylidene; 1,4,7,10,13-pentaazacyclopentadec-1,4-ylene; 1,4,7,10,13-pentaazacyclopentadec-1,7-ylene; 1,4,7,10,13-pentaazacyclopentadec-2,3-ylene; 1,4,7,10,13-pentaazacyclopentadec-1,2-ylene; 1,4,7,10,13-pentaazacyclopentadec-2,2-ylidene; 1,4-diaza-7-thia-cyclonon-1,4-ylene; 1,4-diaza-7-thia-cyclonon-1,2-ylene; 1,4-diaza-7-thia-cyclonon-2,3-ylene; 1,4-diaza-7-thia-cyclonon-6,8-ylene; 1,4-diaza-7-thia-cyclonon-2,2-ylidene; 1,4-diaza-7-oxa-cyclonon-1,4-ylene; 1,4-diaza-7-oxa-cyclonon-1,2-ylene; 1,4-diaza-7-oxa-cyclonon-2,3-ylene; 1,4-diaza-7-oxa-cyclonon-6,8-ylene; 1,4-diaza-7-oxa-cyclonon-2,2-ylidene; 1,4-dioxan-2,3-ylene; 1,4-dioxan-2,6-ylene; 1,4-dioxan-2,2-ylidene; tetrahydropyran-2,3-ylene; tetrahydropyran-2,6-ylene; tetrahydropyran-2,5-ylene; tetrahydropyran-2,2-ylidene; 1,4,7-trithia-cyclonon-2,3-ylene; 1,4,7-trithia-cyclonon-2,9-ylene; and 1,4,7-trithia-cyclonon-2,2-ylidene,

amine: the group $-N(R)_2$ wherein each R is independently selected from: hydrogen; C1-C6-alkyl; C1-C6-alkyl-C6H5; and phenyl, wherein when both R are C1-C6-alkyl both R together may form an -NC3 to an -NC5 heterocyclic ring with any remaining alkyl chain forming an alkyl substituent to the heterocyclic ring,

halogen: selected from the group consisting of: F; Cl; Br and I,

sulfonate: the group $-S(O)_2OR$, wherein R is selected from: hydrogen; C1-C6-alkyl; phenyl; C1-C6-alkyl-C6H5; Li; Na; K; Cs; Mg; and Ca,

sulfate: the group $-OS(O)_2OR$, wherein R is selected from: hydrogen; C1-C6-alkyl; phenyl; Cl-C6-alkyl-C6H5; Li; Na; K; Cs; Mg; and Ca,

sulfone: the group $-S(O)_2R$, wherein R is selected from: hydrogen; C1-C6-alkyl; phenyl; C1-C6-alkyl-C6H5 and amine (to give sulfonamide) selected from the group: -NR'2, wherein each R' is independently selected from: hydrogen; C1-C6-alkyl; C1-C6-alkyl-C6H5; and phenyl, wherein when both R' are C1-C6-alkyl both R' together may form an -NC3 to an - NC5 heterocyclic ring with any remaining alkyl chain forming an alkyl substituent to the heterocyclic ring,

carboxylate derivative: the group -C(O)OR, wherein R is selected from: hydrogen; C1-C6-alkyl; phenyl; C1-C6-alkyl-C6H5; Li; Na; K; Cs; Mg; and Ca,

carbonyl derivative: the group -C(O)R, wherein R is selected from: hydrogen; C1-C6-alkyl; phenyl; C1-C6-alkyl-C6H5 and amine (to give amide) selected from the group: - NR'2, wherein each R' is independently selected from: hydrogen; C1-C6-alkyl; C1-C6-alkyl-C6H5; and phenyl, wherein when both R' are C1-C6-alkyl both R' together may form an - NC3 to an -NC5 heterocyclic ring with any remaining alkyl chain forming an alkyl substituent to the heterocyclic ring,

phosphonate: the group $-P(O)(OR)_2$, wherein each R is independently selected from: hydrogen; C1-C6-alkyl; phenyl; C1-C6-alkyl-C6H5; Li; Na; K; Cs; Mg; and Ca,

phosphate: the group $-OP(O)(OR)_2$, wherein each R is independently selected from: hydrogen; C1-C6-alkyl; phenyl; C1-C6-alkyl-C6H5; Li; Na; K; Cs; Mg; and Ca,

phosphine: the group $-P(R)_2$, wherein each R is independently selected from: hydrogen; C1-C6-alkyl; phenyl; and C1-C6-alkyl-C6H5,

phosphine oxide: the group $-P(O)R_2$, wherein R is independently selected from: hydrogen; C1-C6-alkyl; phenyl; and C1-C6-alkyl-C6H5; and amine (to give phosphonamidate) selected from the group: -NR'2, wherein each R' is independently selected from: hydrogen; C1-C6-alkyl; C1-C6-alkyl-C6H5; and phenyl, wherein when both R' are C1-C6-alkyl both R' together may form an -NC3 to an -NC5 heterocyclic ring with any remaining alkyl chain forming an alkyl substituent to the heterocyclic ring.

Unless otherwise specified the following are more preferred group restrictions that may be applied to groups found within compounds disclosed herein:

alkyl: linear and branched C1-C6-alkyl,

alkenyl: C3-C6-alkenyl,

cycloalkyl: C6-C8-cycloalkyl,

alkoxy: C1-C4-alkoxy,

alkylene: selected from the group consisting of: methylene; 1,2-ethylene; 1,3-propylene; butan-2-ol-1,4-diyl; 1,4-butylene; cyclohexane-1,1-diyl; cyclohexan-1,2-diyl; cyclohexan-1,4-diyl; cyclopentane-1,1-diyl; and cyclopentan-1,2-diyl,

aryl: selected from group consisting of: phenyl; biphenyl; naphthalenyl; anthracenyl; and phenanthrenyl,

arylene: selected from the group consisting of: 1,2-phenylene; 1,3-phenylene; 1,4-phenylene; 1,2-naphtalenylene; 1,4-naphtalenylene; 2,3-naphtalenylene and 1-hydroxy-2,6-phenylene,

heteroaryl: selected from the group consisting of: pyridinyl; pyrimidinyl; quinolinyl; pyrazolyl; triazolyl; isoquinolinyl; imidazolyl; and oxazolidinyl, wherein the heteroaryl may be connected to the compound via any atom in the ring of the selected heteroaryl,

heteroarylene: selected from the group consisting of: pyridin-2,3-diyl; pyridin-2,4-diyl; pyridin-2,6-diyl; pyridin-3,5-diyl; quinolin-2,3-diyl; quinolin-2,4-diyl; isoquinolin-1,3-diyl; isoquinolin-1,4-diyl; pyrazol-3,5-diyl; and imidazole-2,4-diyl,

heterocycloalkyl: selected from the group consisting of: pyrrolidinyl; morpholinyl; piperidinyl; piperidinyl; 1,4-piperazinyl; tetrahydrofuranyl; 1,4,7-triazacyclononanyl; 1,4,8,11-tetraazacyclotetradecanyl; 1,4,7,10,13-pentaazacyclopentadecanyl; 1,4,7,10-tetraazacyclododecanyl; and piperazinyl, wherein the heterocycloalkyl may be connected to the compound via any atom in the ring of the selected heterocycloalkyl,

heterocycloalkylene: selected from the group consisting of: piperidin-2,6-ylene; piperidin-4,4-ylidene; 1,4-piperazin-1,4-ylene; 1,4-piperazin-2,3-ylene; 1,4-piperazin-2,6-ylene; tetrahydrothiophen-2,5-ylene; tetrahydrothiophen-3,4-ylene; tetrahydrofuran-2,5-ylene; tetrahydrofuran-3,4-ylene; pyrrolidin-2,5-ylene; pyrrolidin-2,2-ylidene; 1,4,7-triazacyclonon-1,4-ylene; 1,4,7-triazacyclonon-2,3-ylene; 1,4,7-triazacyclonon-2,2-ylidene; 1,4,8,11-tetraazacyclotetradec-1,4-ylene; 1,4,8,11-tetraazacyclotetradec-1,8-ylene; 1,4,8,11-tetraazacyclotetradec-2,3-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-2,3-ylene; 1,4,7,10-tetraazacyclododec-2,2-ylidene; 1,4,7,10,13-pentaazacyclopentadec-1,4-ylene; 1,4,7,10,13-pentaazacyclopentadec-1,7-ylene; 1,4-diaza-7-thia-cyclonon-1,4-ylene; 1,4-diaza-7-thia-cyclonon-2,3-ylene; 1,4-diaza-7-thia-cyclonon-2,2-ylidene; 1,4-diaza-7-oxa-cyclonon-1,4-ylene; 1,4-diaza-7-oxa-cyclonon-2,3-ylene;1,4-diaza-7-oxa-cyclonon-2,2-ylidene; 1,4-dioxan-2,6-ylene; 1,4-dioxan-2,2-ylidene; tetrahydropyran-2,6-ylene; tetrahydropyran-2,5-ylene; and tetrahydropyran-2,2-ylidene,

amine: the group $-N(R)_2$, wherein each R is independently selected from: hydrogen; C1-C6-alkyl; and benzyl,

halogen: selected from the group consisting of: F and Cl,

sulfonate: the group $-S(O)_2OR$, wherein R is selected from: hydrogen; C1-C6-alkyl; Na; K; Mg; and Ca,

sulfate: the group $-OS(O)_2OR$, wherein R is selected from: hydrogen; C1-C6-alkyl; Na; K; Mg; and Ca,

sulfone: the group $-S(O)_2R$, wherein R is selected from: hydrogen; C1-C6-alkyl; benzyl and amine selected from the group: -NR'2, wherein each R' is independently selected from: hydrogen; C1-C6-alkyl; and benzyl,

carboxylate derivative: the group -C(O)OR, wherein R is selected from hydrogen; Na; K; Mg; Ca; C1-C6-alkyl; and benzyl,

carbonyl derivative: the group: -C(O)R, wherein R is selected from: hydrogen; C1-C6-alkyl; benzyl and amine selected from the group: -NR'2, wherein each R' is independently selected from: hydrogen; C1-C6-alkyl; and benzyl,

phosphonate: the group $-P(O)(OR)_2$, wherein each R is independently selected from: hydrogen; C1-C6-alkyl; benzyl; Na; K; Mg; and Ca,

phosphate: the group $-OP(O)(OR)_2$, wherein each R is independently selected from: hydrogen; C1-C6-alkyl; benzyl; Na; K; Mg; and Ca,

phosphine: the group $-P(R)_2$, wherein each R is independently selected from: hydrogen; C1-C6-alkyl; and benzyl,

phosphine oxide: the group $-P(O)R_2$, wherein R is independently selected from: hydrogen; C1-C6-alkyl; benzyl and amine selected from the group: -NR'2, wherein each R' is independently selected from: hydrogen; C1-C6-alkyl; and benzyl.

The invention will now be further illustrated by way of the following non-limiting examples:

EXAMPLES

The following compounds were prepared and tested for catalytic bleaching activity using air:

Compound 1: $[Fe(L^1)]\{FeCl_4\}Cl$
$L^1$=1,4,7-tris(pyrazol-3-ylmethyl)-1,4,7-triazacyclononane

Compound 2: $[Fe(L^2)]\{FeCl_4\}Cl$
$L^2$=1,4,7-tris(pyrazol-1-ylmethyl)-1,4,7-triazacyclononane

Compound 3: $[FeL^3Br]ClO_4$
$L^3$=1,4-bis(quinolin-2-ylmethyl)-7-ethyl-1,4,7-triazacyclononane

Compound 4: $[FeL^4Cl](ClO_4)_2$
$L^4$=1,4-bis(pyridin-2-ylmethyl)-7-ethyl-1,4,7-triazacyclononane

Compound 5: $[FeL^5Br]BPh_4$
$L^5$=1,4-bis(pyrazol-1-ylmethyl)-7-ethyl-1,4,7-triazacyclononane

(Compounds 1 and 2 for these studies were received from Prof. F. Mani, University of Florence, Florence, Italy, hereby gratefully acknowledged).

Syntheses:

Synthesis of starting materials:

*1,4,7-triazacyclononane*

Ligand 1,4,7-triazacyclononane was produced according the modified method used by the team of Prof. Wieghardt . In this method the detosylation of the 1,4,7-tris-p-toluenesulfon-1,4,7-triazacylononanamide is performed in 5 minutes in hot sulphuric acid of 180°C. Once the solution has cooled down it is transferred into ether under vigorous stirring. The solution that surfaces is decanted and the residue is dissolved in some boiling water. At boiling temperature drops of concentrated hydrochloric acid are added. The brown crystals that precipitate are drained off and washed with cold hydrochloric acid and then with ethanol and ether. The 1,4,7-triazacyclononane trihydrochloride thus produced is then processed further as described by Wieghardt et al (K. Wieghardt et al, Chem Ber., **112**, 2200 (1979)).

*1,4,7-triazatricyclo[5.2.1.0$^{410}$]decane (orthoamide)*

0.5 mol 1,4,7-triazacyclononane, 64.3 g, 0.54 mol orthoformicacidtriethylester, 74.8 g, and 20 mmol p-toluolsulphonacid, 4 g, are heated to 150°C. The ethanol that is created and some of the esters are distilled off. After the reaction has been completed the orthoamide can be distilled off at a pressure of <80 mbar in the form of a bright yellow

volatile oil (b.p. 350 K at 133 Pa), in agreement with literature (T.J. Atkins, *J. Am. Chem. Soc.*, **102,** 6365 (1980)).

*1-ethyl-1,4,7-triazacyclononane (Et-tacn)*

Into a mixture of 0.1 mol orthoamide, 13.92 g, dissolved in dry THF, slowly 0.1 mol ethylbromide, 10.9 g, is dripped. The suspension is stirred for 2 days at room temperature in a closed flask. The microcrystalline powder is drained off and washed with some dry THF. The resulting bromide salt is very hygroscopic. The salt is dissolved in 80 ml water and boiled for 4 hours under back-flow. Then 16 g sodium hydroxide dissolved in 20 ml water is added. This creates a 4 molar reaction mixture. Immediately, a bright yellow oil is separated. To complete the reaction, boiling is continued for another 20 hours. After cooling down 300 ml toluol is added and the water is distilled off by means of a water separator. The reaction mixture is filtered and the toluol is drained off by a rotary evaporator. The remaining product is a bright yellow oil. Yield: 13.8 g (89%). $^1$H-NMR ($CDCl_3$ 270 MHz; 300K): 2.59-2.39 (m; 14H); 1.83 (s, 2H); 0.90 ppm (t; 3H); $^{13}$C-NMR: 52.1; 50.7; 46.5; 46.4; 12.4 ppm.

Compound 3: [Fe(1,4-bis(quinolin-2-ylmethyl)-7-ethyl-1,4,7-triazacyclononane)Br]($ClO_4$) :

*Quinolin-2-ylmethylbromide*

The quinolinemethylbromide is produced as follows. In this method 0.2 mol quinoline (30.0 g) with 0.22 mol N-bromsuccinimid (42 g) and dibenzoylperoxide as starter are placed in 300 ml freshly distilled benzene under irradiation of light. The succinimid that is sedimented after strong cooling is filtered off and the benzene is rotated off. The remaining oil is put into 5% hydrobromic acid. Under cooling with ice a saturated solution of sodiumcarbonate is added to the watery solution up to a pH-value of 7. The precipitated yellowish product is drained off and recrystallized from pentane.

*1,4-bis(quinolin-2-ylmethyl)-7-ethyl-1,4,7-triazacyclononane ($L^3$)*

20 mmol Et-tacn (3.12 g) is dissolved in 50 ml dry THF and diluted with 8 ml triethylamine (56.8 mmol). Then 40 mmol quinolin-2ylmethylbromide (8.96 g) is added, after which the solution turns brown. The reaction mixture is stirred for 3 days. The resulting triethylammoniumbromide is filtered off and the THF is rotated off. What remains is a red to brown oil. The by-products (approx. 8%) created by the alkaline hydrolysis of the chinolylmethylbromide could not be separated by HPLC, GC or chromatography, the ligand analysed.
Yield: 6.6 g (75%). $^1$H-NMR ($CDCl_3$- 400 MHz; 300K): 7.92 (d;2H); 7.89 (d;2H); 7.62 (d;2H); 7.52 (d;2H); 7.50 (m;2H); 7.34 (m;2H); 3.87 (s;4H); 2.94 (m;4H); 2.88 (m;4H); 2.68 (m;4H); 2.53 (q;2H); 0.92 ppm (t; 3H); $^{13}$C-NMR: 160.2; 147.1; 135.9; 129.0; 128.5; 127.2; 127.0; 125.8; 121.1; 64.9; 55.3; 54.3; 53.6; 51.1; 11.8 ppm. MS (EI): 439 ($M^+$; rel int 20%; 157 (rel int. 40% - quinoline-2carboxaldehyde); 143 (rel int 100%-quinoline).

*[Fe(1,4-bis(quinolin-2-ylmethyl)-7-ethyl-1,4,7-triazacyclononane)Br]($ClO_4$):*

Dissolve 1 mmol 1,4-bis(quinolin-2-ylmethyl)-7-ethyl-1,4,7-triazacyclononane, 0.44 g, in 30 ml methanol (bright yellow) and lead through argon. Add 1 mmol $FeBr_2$ (0.22) g. Heat the reaction mixture for 2 hours under back-flow and argon atmosphere. An orange solution is produced. The solution is filtered via an argon frit under protective gas atmosphere to remove undissolved iron bromide. Sodium perchlorate is added to the filtrate and stirred for 2 hours at room temperature. An orange solid is produced. This can be drained off quickly by air and washed with ether. The product is air-stable.
Yield: 400 mg (59%). Elem. Anal. Found: C: 48.24; H: 4.63; N: 10.02%. Calc.: C: 49.85; H: 4.89; N: 10.38%

Compound 4: [Fe(1,4-bis(pyridyl-2-methyl)-7-ethyl-1,4,7-triazacyclononane)Cl]($ClO_4$)$_2$:

*1,4-bis(pyridyl-2-methyl)-7-ethyl-1,4,7-triazacyclononane ($L^4$)*

7.76 g Et-tacn (50 mmol) is suspended in 120 ml water, then 16.4 g picolylhydrochloride (100 mmol) is added, after which the solution turns yellow. Under cooling with ice 8.0 g NaOH is added in portions over a period of 5 days in such a way that the pH-value remains below 9 and the temperature does not exceed 0°C. The solution gradually becomes red to brown. The solution is put in the refrigerator for one day. Any organic phase that has formed is separated. The watery phase is extracted by repeated shaking with chloroform. The combined organic phases are dried over CaO. The chloroform is rotated off and a thick, mostly red-brown oil remains. This oil is still contaminated by traces of picolylchloride and by-products of the alkaline hydrolysis of the picolylchlorides (approx. 5%). A further purification without analysis of the ligand $L^4$ by HPLC, GC or chromatography was not possible. Yield: 14.3 g (84%) $^1$H-NMR ($CDCl_3$- 400 MHz; 300K) :

8.34 (d; 2H); 7.47 (m; 2H); 7.31 (d; 2H); 6.97 (m; 2H) ; 3.68 (s; 4H); 2.78(m; 4H); 2.73 (m; 4H); 2.67 (m; 4H); 2.49 (q; 2H); 0.90 ppm (t; 3H); $^{13}$C-NMR: 159.8; 145.6; 140.0; 123.0; 121.5; 63.8; 55.8; 55.0; 54.3; 51.7; 12.2 ppm. MS (EI): m/z: 339.

*[FeL$^2$Cl] ($ClO_4$)$_2$*

The iron complex was prepared in analogous manner to the formation of the complex for Compound 3.

Compound 5: [Fe(1,4-bis(pyrazol-1-ylmethyl)-7-ethyl-1,4,7-triazacyclononane)Br]($BPh_4$):

*1,4-bis(pyrazol-1-ylmethyl)-7-ethyl-1,4,7-triazacyclononane ($L^5$)*

The ligand can be synthesised by heating 20 mmol Et-tacn - (3.10 g), 40 mmol pyrazolylmethanol (3.92) (ref W. Driessen, Recl. Trav., Chim. Pays-Bas, **101,** 441, 1982) and 0.4 g LiOH in 50 ml acetonitril for 20 hours under back-flow and argon atmosphere. The solution is filtered and the solvent is rotated off. The product has the form of a bright yellow oil. Yield: 6.3 g (80%). $^1$H-NMR ($CDCl_3$- 400 MHz; 300K) : 7.43 (d; 4H); 6.21 (s; 2H) ; 4.93 (s, 4H); 2.83(m; 8H); 2.62 (m; 4H); 2.53 (q; 2H); 0.95 (t, 3H) ; $^{13}$C-NMR: 139.0; 129.3; 125.9; 72.6; 54.3; 53.5; 52.7; 51.7; 12.3 ppm. MS (EI): m/z: 317.

*[Fe(1,4-bis(pyrazol-1-ylmethyl)-7-ethyl-1,4,7-triazacyclononane)Br] ($BPh_4$):*

1 mmol $FeBr_2$, 0.22 g, is dissolved in oxygen-free ethanol under boiling. 1 mmol 1,4-bis(pyrazol-1-ylmethyl)-7-ethyl-1,4,7-triazacyclononane (0.32 g) is dissolved in 30 ml ethanol (bright yellow) and led through Ar. The ligand solution is then added in drops. After one hour sodium tetraphenylborate in oxygen-free acetone is added in drops and immediately a bright solid is formed. This is stirred for approx. another 2 hours in an argon atmosphere. The solid is quickly drained off in air and washed repeatedly with ether. The white solid is air-stable. Yield: 480 mg (62%). Elem. Anal. Found: C: 61.95; H: 6.80; N: 12.48%. Calc.: C: 62.18; H: 6.09; N: 12.70%

Ligand $L^6$: 1,4-bis(3,5-dimethvipyrazol-1-ylmethyl)-7-ethyl-1,4,7-triazacyclononane:

This ligand can be produced by heating 3.10 g Et-tacn (20 mmol), 5.13 g 3,5-dimethylpyrazol-1-ylmethanol (40 mmol)) (ref W. Driessen, Recl. Trav., Chim. Pays-Bas, **101**, 441, 1982) and 0.5 g potassium carbonate in 50 ml-acetonitril under back-flow and argon atmosphere. The solution is filtered and the solvent is rotated off. The product has the form of a bright yellow oil.
Yield: 3.7 g (50%). $^1$H-NMR ($CDCl_3$- 400 MHz; 300K): 5.72 (s; 2H) ; 4.69 (s, 4H); 2.78(m; 8H); 2.58 (m; 4H); 2.46 (q; 2H); 2.20 (s; 6H); 2.13 (s; 6H); 0.93 (t, 3H); $^{13}$C-NMR: 147.0; 139.2; 105.3; 69.6; 54.5; 53.5; 53.0; 51.7; 13.4; 12.6; 11.2 ppm. MS (EI): m/z: 373.

Ligand $L^7$: 1,4-bis(1-methylimidazol-2-ylmethyl)-7-ethyl-1,4,7-triazacyclononane:

*1-methylimidazolyl-1-methanol*

The 1-methylimidazolyl-1-methanol is produced according to a modified iterature procedure (R.C. Jones, J. Am. Chem. Soc., **71**, 383 (1949)). In this method 41.05 g 1-methylimidazol (0.5 mol) and 15.15 g paraformaldehyde (0.5 mol) are heated together in an autoclave for 24 hours at 140°C, during which a pressure of approx. 10 bar develops. The autoclave is allowed to cool down to approx. 90°C and then opened. The reaction mixture is poured into a flask and the autoclave is rinsed with methanol. The methanol is rotated off and the residue is put in ethanol. Next, 75 ml concentrated HCl is added. The reaction mixture is reduced to dry matter. A sticky brown residue remains, that is dissolved in ethanol preferably boiling as little as possible. After some cooling down 400 ml ether is added quickly. A beige-white substance is produced, which is sticky after draining off. The product is dried for several weeks over $P_2O_3$.

*2-chloromethyl-1-methyl-imidazolhydrochloride*

The 2-chloromethyl-1-methyl-imidazolhydrochloride is produced according to the description above. 20 ml thionylchloride is added to a suspension of 5.61 g 1-methylimidazolyl-1-methanol in 5 ml dry benzene. Two phases are built. Stir vigorously for half an hour. Then the combined solvents are rotated off and a bright brown product remains. $^1$H-NMR ($CDCl_3$; 270 MHz): 7.75 (d; 1H); 7.68 (d; 1H); 5.16 (s; 2H); 3.86 (s, 3H); 3.42 (s; 3H). $^{13}$C-NMR: 141.5; 124.7; 119.4; 34.2; 31.7 ppm.

*1,4-bis(1-methylimidazol-2-ylmethyl)-7-ethyl-1,4,7-triazacyclononane*

This ligand is produced through conversion with the 2-chloromethyl-1-methyl-imidazolhydrochloride under impact of bases. 3.32 g of the 2-chloromethyl-1-methyl-imidazolhydrochloride (20 mmol) is suspended in acetonitril whilst cooling with ice. Adding 2.77 ml triethylamine results in a brown solution. After stirring for 10 minutes a white precipitation (triethylammoniumchloride) is formed. This is filtered off and washed with a minimum of acetonitril. 1.55 g Et-tacn (10 mmol) is added to the filtrate and rinsed with acetonitril. Then a further 2.9 ml triethylamine (20 mmol+5% surplus) is added and stirred for 3 hours under an argon atmosphere. Next, the reaction mixture is filtered and the solvents are drained off from the filtrate. The yellow solid product remains. Yield: 3.7 g (50%) ; $^{1}$H-NMR ($CDCl_3$- 250 MHz; 300K): 6.86 (s; 2H) ; 6.85 (s; 2H); 5.27 (s; 4H) ; 3.68 (q; 2H); 3.66 (s; 6H); 3.23 (m; 4H); 2.78 (s; 8H); 1.26 (t, 3H); $^{13}$C-NMR: 145.1; 126.1; 121.7; 51.2-55.2; 33.1; 9.6 ppm. MS (EI): m/z: 345.

<u>Ligand $L^8$: 1,4,7-tris(quinolin-2-ylmethyl)-1,4,7-triazacyclononane:</u>

20 mmol Et-tacn (3.12 g) is dissolved in 50 ml dry THF and mixed with 8 ml triethylamine (56.8 mmol). Then 40 mmol quinolin-2-ylmethylbromide (8.96 g) is added, after which the solution turns brown. The reaction mixture is then stirred for 3 days. The resulting triethylammoniumbromide is filtered off and the THF is rotated off. A bright yellow solid remains. The product is still polluted by approx. 2% triethylamine.
Yield: 7.7 g (70%). %); $^{1}$H-NMR ($CDCl_3$- 250 MHz; 300K) : 8.01 (d; 3H); 7.98 (d; 3H); 7.73 (d; 3H); 7.66 (d; 3H); 7.64 (m; 3H); 7.47 (m; 3H); 4.02 (s; 6H); 2.96 (s; 12H). $^{13}$C-NMR: 160.9; 147.3; 135.9; 129.1; 128.8; 127.4; 127.2; 125.9; 121.3; 65.5; 55.8.

<u>Ligand $L^9$: 1,4-bis(N-methylamido)-7-ethyl-1,4,7-triazacyclononane:</u>

This ligand is produced according to the prescription for the synthesis of amide-functionalised polyazamacrocyles of D. Parker et al (J. Chem. Soc., Perkin Trans, 2, 1990, 1425). 25 mmol 1-ethyl-1,4,7-triazacyclononan, 3.90 g, is dissolved in dried acetonitril and mixed with 50 mmol potassium carbonate, 6.9 g. After adding 50 mmol N-methylbromacetamide (lit W. E. Weaver and W. M. Whaley, J. Am. Chem. Soc., **69**, 515, 1947), 7.60 g, the reaction mixture is heated for 24 hours under an argon atmosphere and back-flow. After cooling down the potassium bromide and the remaining potassium carbonate are filtered off. After the solvent has been removed the product remains as a bright yellow solid. Yield: 6.6 g (75%). $^{1}$H-NMR ($CDCl_3$- 400 MHz; 300K): 8.12 (s; 2H); 3.21 (s; 4H); 2.72 (m; 12H); 2.59 (q, 2H); 1.02 (t; 3H). $^{13}$C-NMR ($CDCl_3$- 270 MHz; 300K): 172.7; 61.5; 56.0; 55.3; 53.7; 52.7; 25.7; 12.0 ppm. MS(EI): m/z: 299.

<u>Ligand $L^{10}$: 1,4-bis(N-isopropylamido)-7-ethyl-1,4,7-triazacyclononane:</u>

25 mmol 1-ethyl-1,4,7-triazacylononan, 3.90 g, is dissolved in dried acetonitril and mixed with 50 mmol potassium carbonate, 6.9 g. After adding 50 mmol N-i-propylbromacetamide (lit W. E. Weaver and W. M. Whaley, J. Am. Chem. Soc., **69**, 515, 1947), 9.0 g, the reaction mixture is heated for 24 hours under an argon atmosphere and back-flow. After cooling down the potassium bromide and the remaining potassium carbonate are filtered off. After the solvent has been removed the product remains as a bright yellow solid, analogous to the description of D. Parker et al. (J. Chem. Soc., Perkin Trans, 2, 1990, 1425).
Yield: 6.2 g (70%) $^{1}$H-NMR ($CDCl_3$- 400 MHz; 300K): 7.35 (d; 2H); 4.01 (sept, 2H); 3.13 (s; 4H); 2.80 (m; 4H); 2.76 (m, 4H); 2.65 (s; 4H); 2.59 (q, 2H); 1.09 (d, 12H); 0.98 (t; 3H). $^{13}$C-NMR ($CDCl_3$- 270 MHz; 300K): 172.7; 62.4; 58.3; 57.6; 55.1; 53.1; 40.8; 22.9; 11.6 ppm.

<u>Experimental:</u>

<u>Example 1:</u>

In an aqueous solution containing 10 mM carbonate buffer (pH 10) without and with 0.6 g/l NaLAS (linear alkylbenzene sulfonate) or containing 10 mM borate buffer (pH 8) without and with 0.6 g/l NaLAS, tomato-soya oil stained cloths were added and kept in contact with the solution under agitation for 30 minutes at 30 °C. In comparative experiments, the same experiments were done by addition of 10 μM complex, referred to in the table below.

After the wash, the cloths were rinsed with water and subsequently dried at 30 °C and the change in colour was measured immediately after drying with a Linotype-Hell scanner (ex Linotype). The change in colour (including bleaching) is expressed as the ΔE value; a higher ΔE value means a cleaner cloth. The measured colour difference (ΔE) between the washed cloth and the unwashed cloth is defined as follows:

$$\Delta E = [(\Delta L)^2 + (\Delta a)^2 + (\Delta b)^2]^{1/2}$$

wherein ΔL is a measure for the difference in darkness between the washed and unwashed test cloth; Δa and Δb are measures for the difference in redness and yellowness respectively between both cloths. With regard to this colour measurement technique, reference is made to Commission International de l'Eclairage (CIE); Recommendation on Uniform Colour Spaces, colour difference equations, psychometric colour terms, supplement no 2 to CIE Publication, no 15, Colormetry, Bureau Central de la CIE, Paris 1978. The results are shown below in Table 1:

Table 1

| | pH 8 - LAS | pH 8 + LAS | pH 10 - LAS | pH 10 + LAS |
|---|---|---|---|---|
| Blank | 1 | 2 | 1 | 3 |
| Compound **1** | 2 | 12 | 1 | 4 |
| Compound **2** | 2 | 14 | 3 | 8 |
| Compound **3** | 16 | 17 | 16 | 17 |
| Compound **4** | 3 | 9 | 3 | 9 |
| Compound **5** | 5 | 10 | 4 | 6 |

Example 2:

Bleach values expressed in ΔE (a higher value means a cleaner cloth). Stain: curry oil stain. Washed for 30 min at 30 °C, rinsed, dried and measured. In all cases 10 μM of metal complex is added to the wash liquor (except for blank). The results are shown below in Table 2:

Table 2

| | pH 8 - LAS | pH 8 + LAS | pH 10 - LAS | pH 10 + LAS |
|---|---|---|---|---|
| Blank | 1 | 3 | 3 | 15 |
| Compound **1** | 2 | 12 | 1 | 24 |
| Compound **2** | 2 | 14 | 3 | 32 |
| Compound **3** | 16 | 17 | 16 | 27 |
| Compound **4** | 3 | 9 | 3 | 23 |
| Compound **5** | 5 | 10 | 4 | 23 |

**Claims**

**1.** A bleaching composition comprising, in an aqueous medium, atmospheric oxygen and a ligand which forms a complex with a transition metal, the complex catalysing bleaching of a substrate by the atmospheric oxygen, wherein the aqueous medium is substantially devoid of peroxygen bleach or a peroxy-based or -generating bleach system, wherein the ligand forms a complex of the general formula (A1):

$$[M_aL_kX_n]Y_m \qquad (A1)$$

in which:

M represents Fe(II)-(III)-(IV)-(V);
X represents a coordinating species selected from any mono, bi or tri charged anions and any neutral molecules able to coordinate the metal in a mono, bi or tridentate manner;
Y represents any non-coordinated counter ion;
a represents an integer from 1 to 10;

k represents an integer from 1 to 10;
n represents an integer from 1 to 10;
m represents zero or an integer from 1 to 20; and
L represents a ligand of the general formula (I), or its protonated or deprotonated analogue:

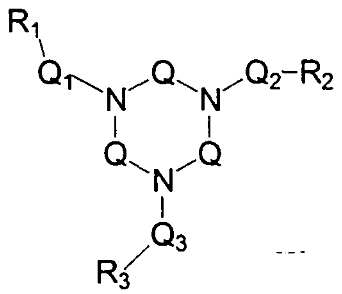

(I)

wherein

R$_1$, R$_2$, and R$_3$ independently represent a group selected from hydrogen, hydroxyl, halogen, -NH-C(NH)NH$_2$, -R and -OR, wherein R= alkyl, alkenyl, cycloalkyl, heterocycloalkyl, aryl, heteroaryl or a carbonyl derivative group, R being optionally substituted by one or more functional groups E;
Q independently represent a group selected from $C_{2-3}$-alkylene optionally substituted by H, benzyl or $C_{1-8}$-alkyl;
Q$_1$, Q$_2$ and Q$_3$ independently represent a group of the formula:

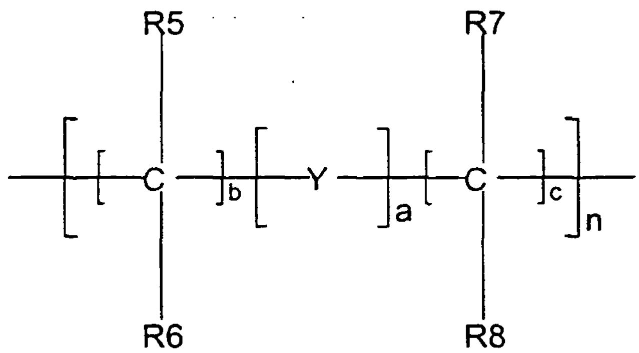

wherein
$5 \geq a+b+c \geq 1$; a=0-5; b=0-5; c=0-5; n=1 or 2;
Y independently represents a group selected from -O-, - S-, -SO-, $-SO_2-$, -C(O)-, arylene, alkylene, heteroarylene, heterocycloalkylene, -(G)P-, -P(O)- and -(G)N- , wherein G is selected from hydrogen, alkyl, aryl, arylalkyl, cycloalkyl, each except hydrogen being optionally substituted by one or more functional groups E;
R5, R6, R7, R8 independently represent a group selected from hydrogen, hydroxyl, halogen, -R and -OR, wherein R represents alkyl, alkenyl, cycloalkyl, heterocycloalkyl, aryl, heteroaryl or a carbonyl derivative group, R being optionally substituted by one or more functional groups E,
or R5 together with R6, or R7 together with R8, or both, represent oxygen,
or R5 together with R7 and/or independently R6 together with R8, or R5 together with R8 and/or independently R6 together with R7, represent $C_{1-6}$-alkylene optionally substituted by $C_{1-4}$-alkyl, -F, -Cl, -Br or -I; and
E independently represents a functional group selected from -F, -Cl, -Br, -I, -OH, -OR', $-NH_2$, -NHR', $-N(R')_2$, $-N(R')_3^+$, -C(O)R', -OC(O)R', -COOH, $-COO^-(Na^+, K^+)$, -COOR', - $C(O)NH_2$, -C(O)NHR', $-C(O)N(R')_2$, heteroaryl, -R', -SR', -SH, - $P(R')_2$, $-P(O)(R')_2$, $-P(O)(OH)_2$, $-P(O)(OR')_2$, $-NO_2$, $-SO_3H$, $-SO_3^-$ $(Na^+, K^+)$, $-S(O)_2R'$, -NHC(O)R', and -N(R')C(O)R', wherein R' represents cycloalkyl, aryl, arylalkyl, or alkyl optionally substituted by -F, -Cl, -Br, -I, $-NH_3^+$, $-SO_3H$, $-SO_3^-(Na^+, K^+)$, -COOH, $-COO^-(Na^+, K^+)$, $-P(O)(OH)_2$, or $-P(O)(O^-(Na^+, K^+))_2$,

provided that at least one, preferably at least two, of $R_1$, $R_2$ and $R_3$ is a coordinating group.

**2.** A bleaching composition according to claim 1, wherein the medium has a pH value in the range from pH 6 to 11, preferably in the range from pH 8 to 10.

**3.** A bleaching composition according to claim 1 or claim 2, wherein the medium is substantially devoid of a transition metal sequestrant.

**4.** A bleaching composition according to any of claims 1 to 3, wherein the medium further comprises a surfactant.

**5.** A bleaching composition according to any of claims 1 to 4, wherein the medium further comprises a builder.

**6.** A bleaching composition according to any of claims 1 to 5, wherein the composition comprises a preformed complex of the ligand and a transition metal.

**7.** A bleaching composition according to any of claims 1 to 5, wherein the ligand is present as a free ligand that complexes with a transition metal present in the water.

**8.** A bleaching composition according to any of claims 1 to 5, wherein the ligand is present as a free ligand that complexes with a transition metal present in the substrate.

**9.** A bleaching composition according to any of claims 1 to 5, wherein the composition comprises the ligand present as a free ligand or a transition metal-substitutable metal-ligand complex, and a source of transition metal.

**10.** A bleaching composition according to any preceding claim, wherein at least two of $R_1$, $R_2$ and $R_3$ independently represent a coordinating group selected from carboxylate, amido, $-NH-C(NH)NH_2$, hydroxyphenyl, an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole.

**11.** A bleaching composition according to any preceding claim, wherein at least two of $R_1$, $R_2$, $R_3$ each independently represent a coordinating group selected from optionally substituted pyridin-2-yl, optionally substituted imidazol-2-yl, optionally substituted imidazol-4-yl, optionally substituted pyrazol-1-yl, and optionally substituted quinolin-2-yl.

**12.** A bleaching composition according to any preceding claim, wherein R5, R6, R7, R8 independently represent a group selected from -H, hydroxy-$C_0$-$C_{20}$-alkyl, halo-$C_0$-$C_{20}$-alkyl, nitroso, formyl-$C_0$-$C_{20}$-alkyl, carboxyl-$C_0$-$C_{20}$-alkyl and esters and salts thereof, carbamoyl-$C_0$-$C_{20}$-alkyl, sulfo-$C_0$-$C_{20}$-alkyl and esters and salts thereof, sulfamoyl-$C_0$-$C_{20}$-alkyl, amino-$C_0$-$C_{20}$-alkyl, aryl-$C_0$-$C_{20}$-alkyl, $C_0$-$C_{20}$-alkyl, alkoxy-$C_0$-$C_8$-alkyl, carbonyl-$C_0$-$C_6$-alkoxy, and $C_0$-$C_{20}$-alkylamide.

**13.** A bleaching composition according to any preceding claim, wherein $Q_1$, $Q_2$ and $Q_3$ are defined such that a=b=0, c=1,2,3 or 4 and n=1.

**14.** A bleaching composition according to any preceding claim, wherein $Q_1$, $Q_2$ and $Q_3$ independently represent a group selected from $-CH_2-$ and $-CH_2CH_2-$.

**15.** A bleaching composition according to any preceding claim, wherein Q represents a group selected from $-CH_2CH_2-$ and $-CH_2CH_2CH_2-$.

**16.** A bleaching composition according to any preceding claim, wherein the ligand L is of the general formula (II):

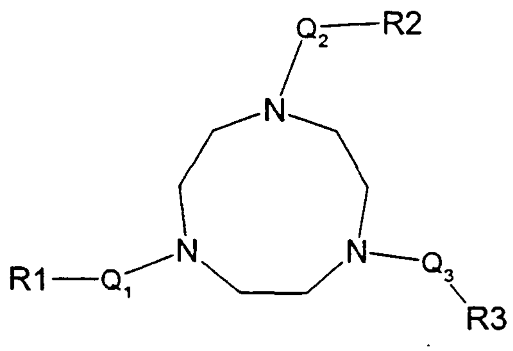

(II)

**17.** A bleaching composition according to claim 16, wherein R1, R2, R3 each independently represent a coordinating group selected from carboxylate, amido, -NH-C(NH)$NH_2$, hydroxyphenyl, an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole.

**18.** A bleaching composition according to claim 17, wherein R1, R2, R3 each independently represent a coordinating group selected from optionally substituted pyridin-2-yl, optionally substituted imidazol-2-yl, optionally substituted imidazol-4-yl, optionally substituted pyrazol-1-yl, and optionally substituted quinolin-2-yl.

**19.** A bleaching composition according to claim 16, wherein
two of R1, R2, R3 each independently represent a coordinating group selected from carboxylate, amido, -NH-C(NH) $NH_2$, hydroxyphenol, an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole; and
one of R1, R2, R3 represents a group selected from hydrogen, $C_{1-20}$ optionally substituted alkyl, $C_{1-20}$ optionally substituted arylalkyl, aryl, and $C_{1-20}$ optionally substituted $NR_3^+$ (wherein R=$C_{1-8}$-alkyl).

**20.** A bleaching composition according to claim 19, wherein
two of R1, R2, R3 each independently represent a coordinating group selected from optionally substituted pyridin-2-yl, optionally substituted imidazol-2-yl, optionally substituted imidazol-4-yl, optionally substituted pyrazol-1-yl, and optionally substituted quinolin-2-yl; and
one of R1, R2, R3 represents a group selected from hydrogen, $C_{1-10}$ optionally substituted alkyl, $C_{1-5}$-furanyl, $C_{1-5}$ optionally substituted benzylalkyl, benzyl, $C_{1-5}$ optionally substituted alkoxy, and $C_{1-20}$ optionally substituted $N^+Me_3$.

**21.** A bleaching composition according to claim 16, wherein L represents a ligand selected from:

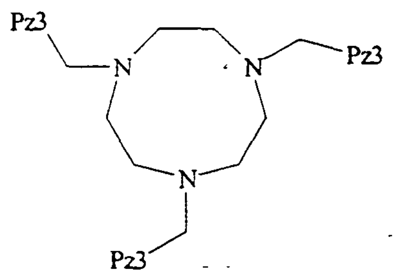

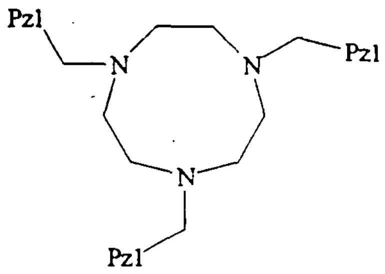

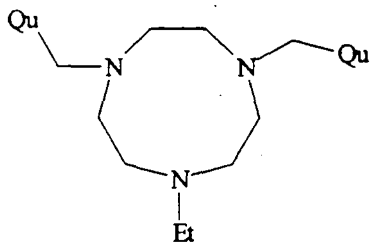

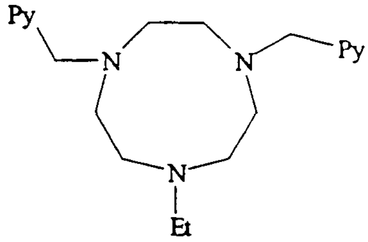

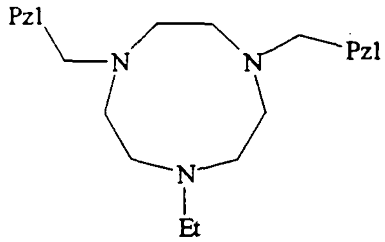

wherein -Et represents ethyl, -Py represents pyridin-2-yl, Pz3 represents pyrazol-3-yl, Pz1 represents pyrazol-1-yl, and Qu represents quinolin-2-yl.

**22.** A bleaching composition according to any preceding claim, wherein the composition comprises a mixture of the ligand L and a metal salt $MX_n$ in which n=1-5, preferably 1-3.

**23.** A method of bleaching a substrate comprising applying to the substrate, in an aqueous medium, a ligand which forms a complex with a transition metal, the complex catalysing bleaching of the substrate by atmospheric oxygen, wherein the ligand is as defined in any of claims 1 to 21.

**24.** A method according to claim 23, wherein the majority of the bleaching species in the medium (on an equivalent weight basis) is derived from the atmospheric oxygen.

**25.** A method according to claim 23 or claim 24, wherein the medium is substantially devoid of peroxygen bleach or a peroxy-based or -generating bleach system.

**26.** A method according to any preceding claim, wherein the aqueous medium is agitated.

**27.** A method according to any of claims 23 to 26, wherein the medium is as defined in any of claims 2 to 5.

**28.** Use of a ligand which forms a complex with a transition metal as a catalytic bleaching agent for a substrate in an aqueous medium substantially devoid of peroxygen bleach or a peroxy-based or -generating bleach system, the complex catalysing bleaching of the substrate by the atmospheric oxygen wherein the ligand is as defined in any of claims 1 to 21.

**29.** A method of treating a textile by contacting the textile with a ligand which forms a complex with a transition metal, whereby the complex catalyses bleaching of the textile by atmospheric oxygen after the treatment, wherein the ligand is as defined in any of claims 1 to 21.

**30.** A method according to claim 29, wherein the treatment comprises contacting the-textile with the ligand in dry form.

**31.** A method according to claim 30, wherein the treatment comprises contacting the textile with a liquor containing the ligand and then drying.

**32.** A method according to claim 31, wherein the liquor is an aqueous liquor.

**33.** A method according to claim 32, wherein the liquor is a spray-on fabric treatment fluid.

**34.** A method according to claim 32, wherein the liquor is a wash liquor for laundry cleaning.

**35.** A method according to claim 31, wherein the liquor is a non-aqueous liquor.

**36.** A method according to claim 35, wherein the liquor is a dry cleaning fluid.

**37.** A method according to claim 35, wherein the liquor is a spray-on aerosol fluid.

**38.** A method according to any of claims 31 to 37, wherein the liquor is substantially devoid of peroxygen bleach or a peroxy-based or -generating bleach system.

**39.** A dry textile having a ligand as defined in any of claims 1 to 21 applied or deposited thereon, whereby bleaching by atmospheric oxygen is catalysed on the textile.

**Patentansprüche**

**1.** Bleichende Zusammensetzung, umfassend in einem wässrigen Medium atmosphärischen Sauerstoff und einen Liganden, der einen Komplex mit einem Übergangsmetall bildet, wobei der Komplex das Bleichen eines Substrats durch den atmosphärischen Sauerstoff katalysiert, wobei das wässrige Medium im Wesentlichen frei von Persauerstoffbleichmittel oder auf Peroxy basierendem oder -erzeugendem Bleichmittelsystem ist,
wobei der Ligand einen Komplex der allgemeinen Formel (A1) bildet:

$$[M_aL_kX_n]Y_m \qquad (A1),$$

worin:

M Fe(II) - (III) - (IV) - (V) wiedergibt;
X eine koordinierende Spezies, ausgewählt aus beliebigen ein-, zwei- oder dreifach geladenen Anionen und beliebigen neutralen Molekülen, die das Metall in einer ein-, zwei- oder dreizähnigen Weise koordinieren können, darstellt;
Y beliebiges, nicht koordiniertes Gegenion wiedergibt;
a eine ganze Zahl von 1 bis 10 wiedergibt;
k eine ganze Zahl von 1 bis 10 wiedergibt;
n eine ganze Zahl von 1 bis 10 wiedergibt;
m null oder eine ganze Zahl von 1 bis 20 wiedergibt; und
L einen Liganden der allgemeinen Formel (I) oder sein protoniertes oder deprotoniertes Analoges wiedergibt:

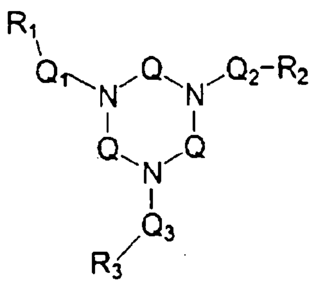

(I)

worin

$R_1$, $R_2$ und $R_3$ unabhängig eine Gruppe, ausgewählt aus Wasserstoff, Hydroxyl, Halogen, $-NH-C(NH)NH_2$, -R und -OR, wiedergeben, worin R= Alkyl-, Alkenyl-, Cycloalkyl-, Heterocycloalkyl-, Aryl-, Heteroaryl- oder eine

Carbonylderivatgruppe, wobei R gegebenenfalls mit einer oder mehreren funktionellen Gruppen E substituiert ist;
Q unabhängig eine Gruppe, ausgewählt aus $C_{2-3}$-Alkylen, gegebenenfalls substituiert mit H, Benzyl oder $C_{1-8}$-Alkyl, wiedergibt;
$Q_1$, $Q_2$ und $Q_3$ unabhängig eine Gruppe der Formel:

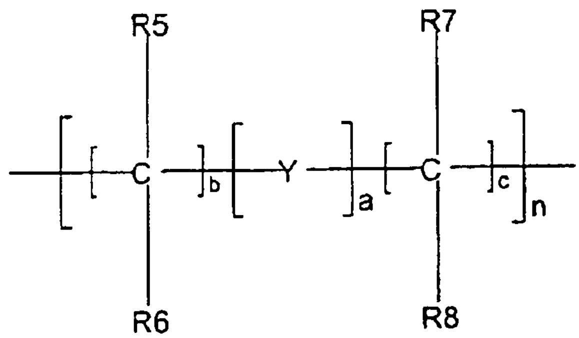

wiedergeben,
worin
$5 \geq a+b+c \geq 1$; a=0-5; b=0-5; c=0-5; n=1 oder 2;

Y unabhängig eine Gruppe, ausgewählt aus -O-, -S-, -SO-, $-SO_2-$, -C(O)-, Arylen, Alkylen, Heteroarylen, Heterocycloalkylen, -(G)P-, -P(O)- und -(G)N-, wiedergibt, worin G aus Wasserstoff, Alkyl, Aryl, Arylalkyl, Cycloalkyl ausgewählt ist, wobei jedes - ausgenommen Wasserstoff - gegebenenfalls mit einer oder mehreren funktionellen Gruppen E substituiert ist;
R5, R6, R7, R8 unabhängig eine Gruppe, ausgewählt aus Wasserstoff, Hydroxyl, Halogen, -R und -OR, wiedergeben, wobei R Alkyl-, Alkenyl-, Cycloalkyl-, Heterocycloalkyl-, Aryl-, Heteroaryl- oder eine Carbonylderivatgruppe wiedergibt, wobei R gegebenenfalls mit einer oder mehreren funktionellen Gruppen E substituiert ist, oder
R5 zusammen mit R6, oder R7 zusammen mit R8, oder beide Sauerstoff wiedergeben, oder
R5 zusammen mit R7, und/oder unabhängig, R6 zusammen mit R8, oder R5 zusammen mit R8, und/oder unabhängig, R6 zusammen mit R7, $C_{1-6}$-Alkylen, gegebenenfalls substituiert mit $C_{1-4}$-Alkyl; -F, -Cl, -Br oder -I, wiedergeben; und
E unabhängig eine funktionelle Gruppe, ausgewählt aus -F, -Cl, -Br, -I, -OH, -OR', $-NH_2$, -NHR', $-N(R')_2$, $-N(R')_3^+$, -C(O)R', -OC(O)R', -COOH, -COO'(Na$^+$, K$^+$), -COOR', $-C(O)NH_2$, -C(0)NHR', $-C(O)N(R')_2$, Heteroaryl, -R', -SR', -SH, $-P(R')_2$, $-P(O)(R')_2$, $-P(O)(OH)_2$, $-P(O)(OR')_2$, $-NO_2$, , $-SO_3H$, $-SO_3^-$(Na$^+$, K$^+$), $-S(O)_2R'$, -NHC(O)R' und -N(R')C(O)R', wiedergibt, worin R' Cycloalkyl, Aryl, Arylalkyl oder Alkyl, gegebenenfalls substituiert mit -F, -Cl, -Br, -I, $-NH_3^+$, $-SO_3H$, $-SO_3^-$ (Na$^+$, K$^+$), -COOH, -COO$^-$(Na$^+$, K$^+$), $-P(O)(OH)_2$ oder -P(O)(O$^-$(Na$^+$, K$^+$))$_2$, wiedergibt,

mit der Maßgabe, dass mindestens einer, vorzugsweise mindestens zwei, von $R_1$, $R_2$ und $R_3$ eine koordinierende Gruppe darstellt/darstellen.

2. Bleichende Zusammensetzung nach Anspruch 1, worin das Medium einen pH-Wert im Bereich von pH 6 bis 11, vorzugsweise im Bereich von pH 8 bis 10, aufweist.

3. Bleichende Zusammensetzung nach Anspruch 1 oder Anspruch 2, worin das Medium im Wesentlichen frei von Übergangsmetallmaskierungsmittel ist.

4. Bleichende Zusammensetzung nach einem der Ansprüche 1 bis 3, worin das Medium weiterhin ein Tensid umfasst.

5. Bleichende Zusammensetzung nach einem der Ansprüche 1 bis 4, worin das Medium weiterhin einen Builder umfasst.

6. Bleichende Zusammensetzung nach einem der Ansprüche 1 bis 5, worin die Zusammensetzung einen vorgebildeten Komplex des Liganden und ein Übergangsmetall umfasst.

7. Bleichende Zusammensetzung nach einem der Ansprüche 1 bis 5, worin der Ligand als ein freier Ligand vorliegt, der mit einem in dem Wasser vorliegenden Übergangsmetall komplexiert.

8. Bleichende Zusammensetzung nach einem der Ansprüche 1 bis 5, worin der Ligand als ein freier Ligand vorliegt, der mit einem in dem Substrat vorliegenden Übergangsmetall komplexiert.

9. Bleichende Zusammensetzung nach einem der Ansprüche 1 bis 5, worin die Zusammensetzung den Liganden umfasst, der als freier Ligand oder ein Übergangsmetall-substituierbarer Metall-Liganden-Komplex oder eine Quelle von Übergangsmetall vorliegt.

10. Bleichende Zusammensetzung nach einem vorangehenden Anspruch, worin mindestens zwei von $R_1$, $R_2$ und $R_3$ unabhängig eine koordinierende Gruppe, ausgewählt aus Carboxylat, Amido, -NH-C(NH)$NH_2$, Hydroxyphenyl, einem gegebenenfalls substituierten heterocyclischen Ring oder einem gegebenenfalls substituierten heteroaromatischen Ring, ausgewählt aus Pyridin, Pyrimidin, Pyrazin, Pyrazol, Imidazol, Benzimidazol, Chinolin, Chinoxalin, Triazol, Isochinolin, Carbazol, Indol, Isoindol, Oxazol und Thiazol, wiedergeben.

11. Bleichende Zusammensetzung nach einem vorangehenden Anspruch, worin mindestens zwei von $R_1$, $R_2$, $R_3$ jeweils unabhängig eine koordinierende Gruppe, ausgewählt aus gegebenenfalls substituiertem Pyridin-2-yl, gegebenenfalls substituiertem Imidazol-2-yl, gegebenenfalls substituiertem Imidazol-4-yl, gegebenenfalls substituiertem Pyrazol-1-yl und gegebenenfalls substituiertem Chinolin-2-yl, wiedergeben.

12. Bleichende Zusammensetzung nach einem vorangehenden Anspruch, worin R5, R6, R7, R8 unabhängig eine Gruppe, ausgewählt aus -H, Hydroxy-$C_0$-$C_{20}$-alkyl, Halogen-$C_0$-$C_{20}$-alkyl, Nitroso, Formyl-$C_0$-$C_{20}$-alkyl, Carboxyl-$C_0$-$C_{20}$-alkyl und Estern und Salzen davon, Carbamoyl-$C_0$-$C_{20}$-alkyl, Sulfo-$C_0$-$C_{20}$-alkyl und Estern und Salzen davon, Sulfamoyl-$C_0$-$C_{20}$-alkyl, Amino-$C_0$-$C_{20}$-alkyl, Aryl-$C_0$-$C_{20}$-alkyl, $C_0$-$C_{20}$-Alkyl, Alkoxy-$C_0$-$C_8$-alkyl, Carbonyl-$C_0$-$C_6$-alkoxy und $C_0$-$C_{20}$-Alkylamid, wiedergeben.

13. Bleichende Zusammensetzung nach einem vorangehenden Anspruch, worin $Q_1$, $Q_2$ und $Q_3$ derart definiert sind, dass a=b=0, c=1, 2, 3 oder 4 und n=1.

14. Bleichende Zusammensetzung nach einem vorangehenden Anspruch, worin $Q_1$, $Q_2$ und $Q_3$ unabhängig eine Gruppe, ausgewählt aus $-CH_2-$ und $-CH_2CH_2-$, wiedergeben.

15. Bleichende Zusammensetzung nach einem vorangehenden Anspruch, worin Q eine Gruppe, ausgewählt aus $-CH_2CH_2-$ und $-CH_2CH_2CH_2-$, wiedergibt.

16. Bleichende Zusammensetzung nach einem vorangehenden Anspruch, worin der Ligand L die allgemeine Formel (II) aufweist:

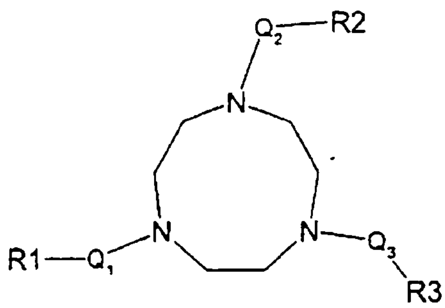

(II)

17. Bleichende Zusammensetzung nach Anspruch 16, worin R1, R2, R3 jeweils unabhängig eine koordinierende Gruppe, ausgewählt aus Carboxylat, Amido, -NH-C(NH)$NH_2$, Hydroxyphenyl, einem gegebenenfalls substituierten heterocyclischen Ring oder einem gegebenenfalls substituierten heteroaromatischen Ring, ausgewählt aus Pyridin, Pyrimidin, Pyrazin, Pyrazol, Imidazol, Benzimidazol, Chinolin, Chinoxalin, Triazol, Isochinolin, Carbazol, Indol, Isoindol, Oxazol und Thiazol, wiedergeben.

**18.** Bleichende Zusammensetzung nach Anspruch 17, worin R1, R2, R3 jeweils unabhängig eine koordinierende Gruppe, ausgewählt aus gegebenenfalls substituiertem Pyridin-2-yl, gegebenenfalls substituiertem Imidazol-2-yl, gegebenenfalls substituiertem Imidazol-4-yl, gegebenenfalls substituiertem Pyrazol-1-yl und gegebenenfalls substituiertem Chinolin-2-yl, wiedergeben.

**19.** Bleichende Zusammensetzung nach Anspruch 16, worin zwei von R1, R2, R3 jeweils unabhängig eine koordinierende Gruppe, ausgewählt aus Carboxylat, Amido, $-NH-C(NH)NH_2$, Hydroxyphenol, einem gegebenenfalls substituierten heterocyclischen Ring oder einem gegebenenfalls substituierten heteroaromatischen Ring, ausgewählt aus Pyridin, Pyrimidin, Pyrazin, Pyrazol, Imidazol, Benzimidazol, Chinolin, Chinoxalin, Triazol, Isochinolin, Carbazol, Indol, Isoindol, Oxazol und Thiazol, wiedergeben; und einer von R1, R2, R3 eine Gruppe, ausgewählt aus Wasserstoff, $C_{1-20}$ gegebenenfalls substituiertem Alkyl, $C_{1-20}$ gegebenenfalls substituiertem Arylalkyl, Aryl und $C_{1-20}$ gegebenenfalls substituiertem $NR_3^+$ (worin R=$C_{1-8}$-Alkyl), wiedergeben.

**20.** Bleichende Zusammensetzung nach Anspruch 19, worin zwei von R1, R2, R3 jeweils unabhängig eine koordinierende Gruppe, ausgewählt aus gegebenenfalls substituiertem Pyridin-2-yl, gegebenenfalls substituiertem Imidazol-2-yl, gegebenenfalls substituiertem Imidazol-4-yl, gegebenenfalls substituiertem Pyrazol-1-yl und gegebenenfalls substituiertem Chinolin-2-yl, wiedergeben; und einer von R1, R2, R3 eine Gruppe, ausgewählt aus Wasserstoff, $C_{1-10}$ gegebenenfalls substituiertem Alkyl, $C_{1-5}$-Furanyl, $C_{1-5}$ gegebenenfalls substituiertem Benzylalkyl, Benzyl, $C_{1-5}$ gegebenenfalls substituiertem Alkoxy und $C_{1-20}$ gegebenenfalls substituiertem $N^+Me_3$, wiedergeben.

**21.** Bleichende Zusammensetzung nach Anspruch 16, worin L einen Liganden wiedergibt, ausgewählt aus:

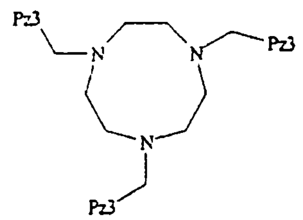

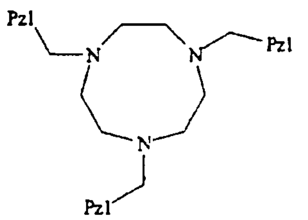

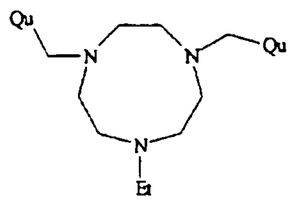

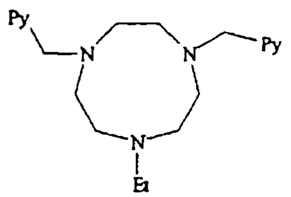

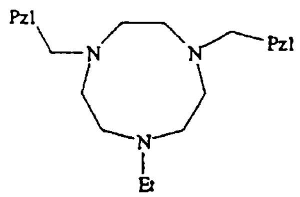

worin -Et Ethyl wiedergibt, -Py Pyridin-2-yl wiedergibt, Pz3 Pyrazol-3-yl wiedergibt, Pz1 Pyrazol-1-yl wiedergibt und Qu Chinolin-2-yl wiedergibt.

**22.** Bleichende Zusammensetzung nach einem vorangehenden Anspruch, worin die Zusammensetzung ein Gemisch von dem Liganden L und einem Metallsalz $MX_n$, worin n=1-5, vorzugsweise 1-3, umfasst.

**23.** Verfahren zum Bleichen eines Substrats, umfassend Auftragen auf das Substrat in einem wässrigen Medium eines Liganden, der einen Komplex mit einem Übergangsmetall bildet, wobei der Komplex das Bleichen des Substrats durch atmosphärischen Sauerstoff katalysiert, wobei der Ligand wie in einem der Ansprüche 1 bis 21 definiert ist.

**24.** Verfahren nach Anspruch 23, wobei die Mehrheit der bleichenden Spezies in dem Medium (oder auf einer Äquivalentgewichtsbasis) von dem atmosphärischen Sauerstoff abgeleitet ist.

**25.** Verfahren nach Anspruch 23 oder Anspruch 24, wobei das Medium im Wesentlichen frei von Persauerstoffbleichmittel oder einem auf Peroxy basierenden oder -erzeugenden Bleichmittelsystem ist.

**26.** Verfahren nach einem vorangehenden Anspruch, wobei das wässrige Medium bewegt wird.

**27.** Verfahren nach einem der Ansprüche 23 bis 26, wobei das Medium wie in einem der Ansprüche 2 bis 5 definiert ist.

**28.** Verwendung eines Liganden, der einen Komplex mit einem Übergangsmetall als ein katalytisches Bleichmittel für ein Substrat in einem wässrigen Medium, das im Wesentlichen frei von Persauerstoffbleichmittel oder einem auf Peroxy basierenden oder -erzeugenden Bleichmittelsystem ist, bildet, wobei der Komplex das Bleichen des Substrats durch den atmosphärischen Sauerstoff katalysiert, wobei der Ligand wie in einem der Ansprüche 1 bis 21 definiert ist.

**29.** Verfahren zum Behandeln eines Textils durch In-Kontakt-Bringen des Textils mit einem Liganden, der einen Komplex mit einem Übergangsmetall bildet, wobei der Komplex das Bleichen des Textils durch atmosphärischen Sauerstoff nach der Behandlung katalysiert, wobei der Ligand wie in einem der Ansprüche 1 bis 21 definiert ist.

**30.** Verfahren nach Anspruch 29, wobei die Behandlung In-Kontakt-Bringen des Textils mit dem Liganden in trockener Form umfasst.

**31.** Verfahren nach Anspruch 30, wobei die Behandlung In-Kontakt-Bringen des Textils mit einer den Liganden enthaltenden Flüssigkeit und dann Trocknen umfasst.

**32.** Verfahren nach Anspruch 31, wobei die Flüssigkeit eine wässrige Flüssigkeit ist.

**33.** Verfahren nach Anspruch 32, wobei die Flüssigkeit ein aufzusprühendes Textilbehandlungsfluid ist.

**34.** Verfahren nach Anspruch 32, wobei die Flüssigkeit eine Waschlauge zum Wäschereinigen ist.

**35.** Verfahren nach Anspruch 31, wobei die Flüssigkeit eine nicht wässrige Flüssigkeit ist.

**36.** Verfahren nach Anspruch 35, wobei die Flüssigkeit ein Trockenreinigungsfluid ist.

**37.** Verfahren nach Anspruch 35, wobei die Flüssigkeit ein aufzusprühendes Aerosolfluid ist.

**38.** Verfahren nach einem der Ansprüche 31 bis 37, wobei die Flüssigkeit im Wesentlichen frei von Persauerstoffbleichmittel oder einem auf Peroxy basierenden oder -erzeugenden Bleichmittelsystem ist.

**39.** Trockenes Textil mit einem wie in einem der Ansprüche 1 bis 21 definierten Liganden, aufgetragen oder darauf abgeschieden, wodurch das Bleichen durch atmosphärischen Sauerstoff auf dem Textil katalysiert wird.

## Revendications

**1.** Composition de blanchiment comprenant, dans un milieu aqueux, de l'oxygène atmosphérique et un ligand qui forme un complexe avec un métal de transition, le complexe catalysant le blanchiment d'un substrat par l'oxygène atmosphérique, dans laquelle le milieu aqueux est sensiblement dépourvu d'agent de blanchiment peroxygéné ou d'un système de blanchiment à base de peroxy ou générant du peroxy,
dans laquelle le ligand forme un complexe de formule générale (A1) :

$$[M_aL_kX_n]\ Y_m \qquad (A1)$$

dans laquelle :

M représente Fe(II)- (III)- (IV)-(V) ;
X représente une espèce de coordination choisie parmi des anions mono, bi ou tri-chargés quelconques et des

molécules neutres quelconques capables de coordonner le métal d'une manière mono, bi ou tridentée ;
Y représente un quelconque contre ion non coordonné ;
a représente un entier de 1 à 10 ;
k représente un entier de 1 à 10 ;
n représente un entier de 1 à 10 ;
m représente zéro ou un entier de 1 à 20 ; et
L représente un ligand de formule générale (I), ou son analogue protoné ou déprotoné :

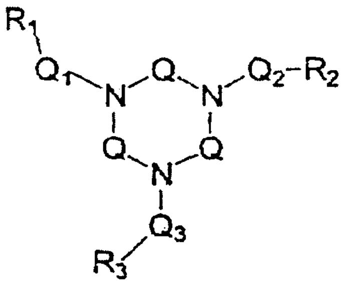

(I)

dans laquelle

R$_1$, R$_2$, et R$_3$ représentent indépendamment un groupe choisi parmi l'hydrogène, l'hydroxyle, l'halogène, -NH-C(NH)NH$_2$, -R et -OR, dans lequel R = alkyle, alcényle, cycloalkyle, hétérocycloalkyle, aryle, hétéroaryle ou un groupe dérivé de carbonyle, R étant facultativement substitué par un ou plusieurs groupes fonctionnels E ;
Q représente indépendamment un groupe choisi parmi un alkylène en C$_2$ à C$_3$ facultativement substitué par H, un benzyle ou un alkyle en C$_1$ à C$_8$ ;
Q$_1$, Q$_2$ et Q$_3$ représentent indépendamment un groupe de formule :

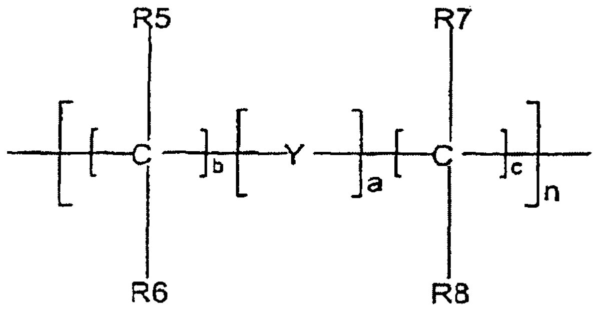

dans laquelle
$5 \geq a + b + c \geq 1$ ; a = 0 à 5 ; b = 0 à 5 ; c = 0 à 5 ; n = 1 ou 2 ;
Y représente indépendamment un groupe choisi parmi -O-, -S-, -SO-, -SO$_2$-, -C(O)-, arylène, alkylène, hétéroarylène, hétérocycloalkylène, -(G)P-, -P(O)- et -(G)N-, dans lequel G est choisi parmi l'hydrogène, l'alkyle, l'aryle, l'arylalkyle, le cycloalkyle, chacun sauf l'hydrogène étant facultativement substitué par un ou plusieurs groupes fonctionnels E ;
R$_5$, R$_6$, R$_7$ et R$_8$ représentent indépendamment un groupe choisi parmi l'hydrogène, l'hydroxyle, l'halogène, -R et -OR, dans lequel R représente un alkyle, un alcényle, un cycloalkyle, un hétérocycloalkyle, un aryle, un hétéroaryle ou un groupe dérivé de carbonyle, R étant facultativement substitué par un ou plusieurs groupes fonctionnels E,
ou R$_5$ conjointement avec R$_6$, ou R$_7$ conjointement avec R$_8$, ou les deux, représentent l'oxygène,
ou R$_5$ conjointement avec R$_7$ et/ou indépendamment R$_6$ conjointement avec R$_8$, ou R$_5$ conjointement avec R$_8$ et/ou indépendamment R$_6$ conjointement avec R$_7$, représentent un alkylène en C$_1$ à C$_6$ facultativement substitué par un alkyle en C$_1$ à C$_4$, -F, -Cl, -Br ou -I ; et

E représente indépendamment un groupe fonctionnel choisi parmi -F, -Cl, -Br, -I, -OH, -OR', $-NH_2$, -NHR', $-N(R')_2$, $-N(R')_3^+$, -C(O)R', -OC (O)R', -COOH, $-COO^-$ ($Na^+$, $K^+$), -COOR', $-C(O)NH_2$, -C(O)NHR', $-C(O)N(R')_2$, un hétéroaryle, -R', -SR', -SH, $-P(R')_2$, $-P(O)(R')_2$, $-P(O)(OH)_2$, $-P(O)(OR')_2$, $-NO_2$, $-SO_3H$, $-SO_3^-$($Na^+$, $K^+$), $-S(O)_2R'$, -NHC(O)R', et -N(R')C(O)R', dans lequel R' représente un cycloalkyle, un aryle, un arylalkyle, ou un alkyle facultativement substitué par -F, -Cl, -Br, -I, $-NH_3^+$, $-SO_3H$, $-SO_3^-$ ($Na^+$, $K^+$), -COOH, $-COO^-$ ($Na^+$, $K^+$), $-P(O)(OH)_2$, ou $-P(O)(O^-(Na^+, K^+))_2$,

sous réserve qu'au moins un, de préférence au moins deux, parmi $R_1$, $R_2$ et $R_3$ soit un groupe de coordination.

**2.** Composition de blanchiment selon la revendication 1, dans laquelle le milieu présente un pH dans la plage de pH 6 à 11, de préférence dans la plage de pH 8 à 10.

**3.** Composition de blanchiment selon la revendication 1 ou la revendication 2, dans laquelle le milieu est sensiblement dépourvu d'un séquestrant de métal de transition.

**4.** Composition de blanchiment selon l'une quelconque des revendications 1 à 3, dans laquelle le milieu comprend en outre un agent tensioactif.

**5.** Composition de blanchiment selon l'une quelconque des revendications 1 à 4, dans laquelle le milieu comprend en outre un adjuvant.

**6.** Composition de blanchiment selon l'une quelconque des revendications 1 à 5, dans laquelle la composition comprend un complexe préformé du ligand et d'un métal de transition.

**7.** Composition de blanchiment selon l'une quelconque des revendications 1 à 5, dans laquelle le ligand est présent sous forme de ligand libre qui se complexe avec un métal de transition présent dans l'eau.

**8.** Composition de blanchiment selon l'une quelconque des revendications 1 à 5, dans laquelle le ligand est présent sous forme d'un ligand libre qui se complexe avec un métal de transition présent dans le substrat.

**9.** Composition de blanchiment selon l'une quelconque des revendications 1 à 5, dans laquelle la composition comprend le ligand présent sous forme d'un ligand libre ou d'un complexe de métal de transition - ligand pouvant être substitué par un métal, et une source de métal de transition.

**10.** Composition de blanchiment selon l'une quelconque des revendications précédentes, dans laquelle au moins deux parmi $R_1$, $R_2$ et $R_3$ représentent indépendamment un groupe de coordination choisi parmi un groupe carboxylate, amido, $-NH-C(NH)NH_2$, hydroxyphényle, un cycle hétérocyclique facultativement substitué ou un cycle hétéroaromatique facultativement substitué choisi parmi la pyridine, la pyrimidine, la pyrazine, le pyrazole, l'imidazole, le benzimidazole, la quinoline, la quinoxaline, le triazole, l'isoquinoline, le carbazole, l'indole, l'isoindole, l'oxazole et le thiazole.

**11.** Composition de blanchiment selon l'une quelconque des revendications précédentes, dans laquelle au moins deux parmi $R_1$, $R_2$ et $R_3$ représentent indépendamment chacun un groupe de coordination choisi parmi le pyridin-2-yle facultativement substitué, l'imidazol-2-yle facultativement substitué, l'imidazol-4-yle facultativement substitué, le pyrazol-1-yle facultativement substitué, et le quinolin-2-yle facultativement substitué.

**12.** Composition de blanchiment selon l'une quelconque des revendications précédentes, dans laquelle $R_5$, $R_6$, $R_7$, $R_8$ représentent indépendamment un groupe choisi parmi -H, hydroxyalkyle en $C_0$ à $C_{20}$, halogénoalkyle en $C_0$ à $C_{20}$, nitroso, formylalkyle en $C_0$ à $C_{20}$, carboxylalkyle en $C_0$ à $C_{20}$ et des esters et des sels de ceux-ci, carbamoylalkyle en $C_0$ à $C_{20}$, sulfoalkyle en $C_0$ à $C_{20}$ et des esters et des sels de ceux-ci, sulfamoylalkyle en $C_0$ à $C_{20}$, aminoalkyle en $C_0$ à $C_{20}$, arylalkyle en $C_0$ à $C_{20}$, alkyle en $C_0$ à $C_{20}$, alcoxyalkyle en $C_0$ à $C_8$, carbonylalcoxy en $C_0$ à $C_6$, et alkylamide en $C_0$ à $C_{20}$.

**13.** Composition de blanchiment selon l'une quelconque des revendications précédentes, dans laquelle $Q_1$, $Q_2$ et $Q_3$ sont définis de telle sorte que a = b = 0, c = 1, 2, 3 ou 4 et n = 1.

**14.** Composition de blanchiment selon l'une quelconque des revendications précédentes, dans laquelle $Q_1$, $Q_2$ et $Q_3$ représentent indépendamment un groupe choisi parmi $-CH_2-$ et $-CH_2CH_2-$.

**15.** Composition de blanchiment selon l'une quelconque des revendications précédentes, dans laquelle Q représente un groupe choisi parmi $-CH_2CH_2-$ et $-CH_2CH_2CH_2-$.

**16.** Composition de blanchiment selon l'une quelconque des revendications précédentes, dans laquelle le ligand L est de formule générale (II) :

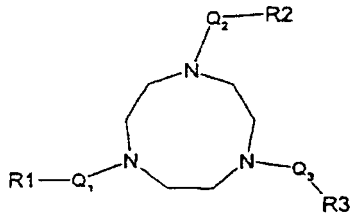

(II)

**17.** Composition de blanchiment selon la revendication 16, dans laquelle $R_1$, $R_2$ et $R_3$ représentent indépendamment chacun un groupe de coordination choisi parmi un groupe carboxylate, amido, $-NH-C(NH)NH_2$, hydroxyphényle, un cycle hétérocyclique facultativement substitué ou un cycle hétéroaromatique facultativement substitué choisi parmi la pyridine, la pyrimidine, la pyrazine, le pyrazole, l'imidazole, le benzimidazole, la quinoline, la quinoxaline, le triazole, l'isoquinoline, le carbazole, l'indole, l'isoindole, l'oxazole et le thiazole.

**18.** Composition de blanchiment selon la revendication 17, dans laquelle $R_1$, $R_2$ et $R_3$ représentent indépendamment chacun un groupe de coordination choisi parmi le pyridin-2-yle facultativement substitué, l'imidazol-2-yle facultativement substitué, l'imidazol-4-yle facultativement substitué, le pyrazol-1-yle facultativement substitué, et le quinolin-2-yle facultativement substitué.

**19.** Composition de blanchiment selon la revendication 16, dans laquelle
deux parmi $R_1$, $R_2$ et $R_3$ représentent indépendamment chacun un groupe de coordination choisi parmi un groupe carboxylate, amide, $-NH-C(NH)NH_2$, hydroxyphénol, un cycle hétérocyclique facultativement substitué ou un cycle hétéroaromatique facultativement substitué choisi parmi la pyridine, la pyrimidine, la pyrazine, le pyrazole, l'imidazole, le benzimidazole, la quinoline, la quinoxaline, le triazole, l'isoquinoline, le carbazole, l'indole, l'isoindole, l'oxazole et le thiazole ; et
un parmi $R_1$, $R_2$, $R_3$ représente un groupe choisi parmi l'hydrogène, un alkyle facultativement substitué en $C_1$ à $C_{20}$, un arylalkyle facultativement substitué en $C_1$ à $C_{20}$, un aryle, et un $NR_3^+$ facultativement substitué en $C_1$ à $C_{20}$ (dans lequel R = alkyle en $C_1$ à $C_8$).

**20.** Composition de blanchiment selon la revendication 19, dans laquelle
deux parmi $R_1$, $R_2$ et $R_3$ représentent indépendamment chacun un groupe de coordination choisi parmi le pyridin-2-yle facultativement substitué, l'imidazol-2-yle facultativement substitué, l'imidazol-4-yle facultativement substitué, le pyrazol-1-yle facultativement substitué, et le quinolin-2-yle facultativement substitué ; et
un parmi $R_1$, $R_2$, $R_3$ représente un groupe choisi parmi l'hydrogène, un alkyle facultativement substitué en $C_1$ à $C_{10}$, un furanyle en $C_1$ à $C_5$, un benzylalkyle facultativement substitué en $C_1$ à $C_5$, un benzyle, un alcoxy facultativement substitué en $C_1$ à $C_5$, et un $N^+Me_3$ facultativement substitué en $C_1$ à $C_{20}$.

**21.** Composition de blanchiment selon la revendication 16, dans laquelle L représente un ligand choisi parmi :

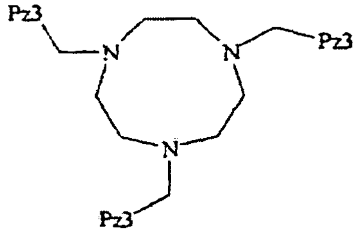

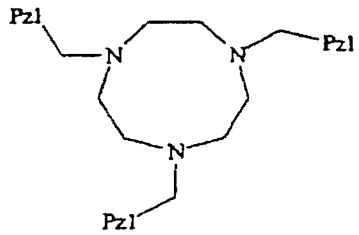
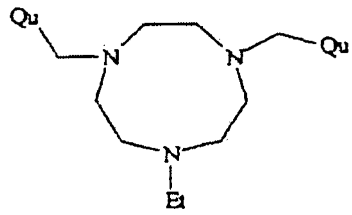

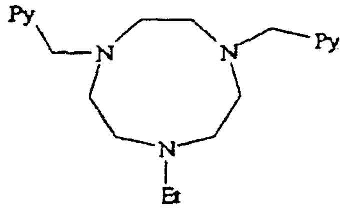
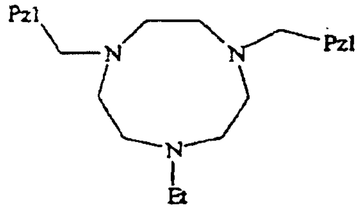

dans lesquelles -Et représente l'éthyle, -Py représente le pyridin-2-yle, Pz3 représente le pyrazol-3-yle, Pz1 représente le pyrazol-1-yle, et Qu représente le quinolin-2-yle.

**22.** Composition de blanchiment selon l'une quelconque des revendications précédentes, dans laquelle la composition comprend un mélange du ligand L et d'un sel métallique $MX_n$ dans lequel n = 1 à 5, de préférence 1 à 3.

**23.** Procédé de blanchiment d'un substrat comprenant l'application sur le substrat, dans un milieu aqueux, d'un ligand qui forme un complexe avec un métal de transition, le complexe catalysant le blanchiment du substrat par l'oxygène atmosphérique, le ligand étant tel que défini selon l'une quelconque des revendications 1 à 21.

**24.** Procédé selon la revendication 23, dans lequel la majorité des espèces de blanchiment dans le milieu (sur une base pondérale équivalente) est dérivée de l'oxygène atmosphérique.

**25.** Procédé selon la revendication 23 ou la revendication 24, dans lequel le milieu est sensiblement dépourvu d'un agent de blanchiment peroxygéné ou d'un système de blanchiment à base de peroxy ou générant du peroxy.

**26.** Procédé selon l'une quelconque des revendications précédentes, dans lequel le milieu aqueux est agité.

**27.** Procédé selon l'une quelconque des revendications 23 à 26, dans lequel le milieu est tel que défini selon l'une quelconque des revendications 2 à 5.

**28.** Utilisation d'un ligand qui forme un complexe avec un métal de transition tel qu'un agent de blanchiment catalytique pour un substrat dans un milieu aqueux sensiblement dépourvu d'agent de blanchiment peroxygéné ou d'un système de blanchiment à base de peroxy ou générant du peroxy, le complexe catalysant le blanchiment du substrat par l'oxygène atmosphérique dans lequel le ligand est tel que défini selon l'une quelconque des revendications 1 à 21.

**29.** Procédé de traitement d'un textile en mettant en contact le textile avec un ligand qui forme un complexe avec un métal de transition, moyennant quoi le complexe catalyse le blanchiment du textile par l'oxygène atmosphérique après le traitement, le ligand étant tel que défini selon l'une quelconque des revendications 1 à 21.

**30.** Procédé selon la revendication 29, dans lequel le traitement comprend la mise en contact du textile avec le ligand sous forme sèche.

**31.** Procédé selon la revendication 30, dans lequel le traitement comprend la mise en contact du textile avec une liqueur contenant le ligand puis un séchage.

**32.** Procédé selon la revendication 31, dans lequel la liqueur est une liqueur aqueuse.

**33.** Procédé selon la revendication 32, dans lequel la liqueur est un liquide de traitement de tissu à pulvériser.

**34.** Procédé selon la revendication 32, dans lequel la liqueur est une liqueur de lavage destinée au blanchissage.

**35.** Procédé selon la revendication 31, dans lequel la liqueur est une liqueur non aqueuse.

**36.** Procédé selon la revendication 35, dans lequel la liqueur est un liquide pour nettoyage à sec.

**37.** Procédé selon la revendication 35, dans lequel la liqueur est un liquide en aérosol à pulvériser.

**38.** Procédé selon l'une quelconque des revendications 31 à 37, dans lequel la liqueur est sensiblement dépourvue d'agent de blanchiment peroxygéné ou d'un système de blanchiment à base de peroxy ou générant du peroxy.

**39.** Textile sec comportant un ligand tel que défini selon l'une quelconque des revendications 1 à 21, appliqué ou déposé sur celui-ci, moyennant quoi le blanchiment par l'oxygène atmosphérique est catalysé sur le textile.